# EUROPEAN PATENT SPECIFICATION

(11) **EP 3 548 081 B1**
(45) Date of publication and mention of the grant of the patent: **10.11.2021**
(21) Application number: 17723305.3
(22) Date of filing: 02.05.2017
(51) Int. Cl.: A61K 39/395, A61K 31/706, A61P 35/00

(54) **METHODS AND COMPOSITIONS FOR THE TREATMENT OF MYELODYSPLASTIC SYNDROME**
VERFAHREN UND ZUSAMMENSETZUNGEN ZUR BEHANDLUNG VON MYELODYSPLASTISCHEM SYNDROM
PROCÉDÉS ET COMPOSITIONS DESTINÉS AU TRAITEMENT DU SYNDROME MYÉLODYSPLASIQUE

(30) Priority: 02.12.2016 US 201662429313 P
(43) Date of publication of application: 09.10.2019
(73) Proprietor: Neuramedy Co., LTD., Seoul (KR)
(72) Inventor: REILLY, Mary, Dublin 2 (IE); MILLER, Robert, Stevenage Hertfordshire SG1 2NY (GB); GARCIA MANERO, Guillermo, Houston Texas 77030 (US)
(74) Representative: Murgitroyd & Company
(86) International application number: PCT/EP2017/060339
(87) International publication number: WO 2018/099614

(56) References cited:
- EP-A1- 2 722 342
- WO-A1-2011/003925
- WO-A2-2013/144345
- Anonymous: "Paper: A Clinical Study of OPN-305, a Toll-like Receptor 2 (TLR-2) Antibody, in Patients with Lower Risk Myelodysplastic Syndromes (MDS) That Have Received Prior Hypomethylating Agent (HMA) Therapy", , 3 December 2016 (2016-12-03), XP055381141, Retrieved from the Internet: URL:https://ash.confex.com/ash/2016/webpro gram/Paper97931.html [retrieved on 2017-06-13]
- Anonymous: "NCT02363491 on 2016_10_11: ClinicalTrials.gov Archive", , 11 October 2016 (2016-10-11), XP055381135, Retrieved from the Internet: URL:https://clinicaltrials.gov/archive/NCT 02363491/2016_10_11 [retrieved on 2017-06-13]
- Anonymous: "Latest news and press releases from Opsona Therapeutics - Ireland", , 22 November 2016 (2016-11-22), XP055381142, Retrieved from the Internet: URL:http://www.opsona.com/index.jsp?p=98&n =154&a=129 [retrieved on 2017-06-13]

### SEQUENCE LISTING

<110> Opsona Therapeutics Limited
<120> Methods and Compositions for the Treatment of Myelodysplastic
   Syndromes
<130> P184609.WO.01
<160> 13
<170> PatentIn version 3.5
<210> 1
   <211> 784
   <212> PRT
   <213> Homo sapiens
<400> 1
<210> 2
   <211> 784
   <212> PRT
   <213> Mus musculus
<400> 2
   Met Leu Arg Ala Leu Trp Leu Phe Trp Ile Leu Val Ala Ile Thr Val
   1               5                   10                  15
   Leu Phe Ser Lys Arg Cys Ser Ala Gln Glu Ser Leu Ser Cys Asp Ala
               20                  25                  30
   Ser Gly Val Cys Asp Gly Arg Ser Arg Ser Phe Thr Ser Ile Pro Ser
           35                  40                  45
   Gly Leu Thr Ala Ala Met Lys Ser Leu Asp Leu Ser Phe Asn Lys Ile
       50                  55                  60
   Thr Tyr Ile Gly His Gly Asp Leu Arg Ala Cys Ala Asn Leu Gln Val
   65                  70                  75                  80
   Leu Met Leu Lys Ser Ser Arg Ile Asn Thr Ile Glu Gly Asp Ala Phe
                   85                  90                  95
   Tyr Ser Leu Gly Ser Leu Glu His Leu Asp Leu Ser Asp Asn His Leu
               100                 105                 110
   Ser Ser Leu Ser Ser Ser Trp Phe Gly Pro Leu Ser Ser Leu Lys Tyr
               115                 120                 125
   Leu Asn Leu Met Gly Asn Pro Tyr Gln Thr Leu Gly Val Thr Ser Leu
       130                 135                 140
   Phe Pro Asn Leu Thr Asn Leu Gln Thr Leu Arg Ile Gly Asn Val Glu
   145                 150                 155                 160
   Thr Phe Ser Glu Ile Arg Arg Ile Asp Phe Ala Gly Leu Thr Ser Leu
                   165                 170                 175
   Asn Glu Leu Glu Ile Lys Ala Leu Ser Leu Arg Asn Tyr Gln Ser Gln
               180                 185                 190
   Ser Leu Lys Ser Ile Arg Asp Ile His His Leu Thr Leu His Leu Ser
           195                 200                 205
   Glu Ser Ala Phe Leu Leu Glu Ile Phe Ala Asp Ile Leu Ser Ser Val
       210                 215                 220
   Arg Tyr Leu Glu Leu Arg Asp Thr Asn Leu Ala Arg Phe Gln Phe Ser
   225                 230                 235                 240
   Pro Leu Pro Val Asp Glu Val Ser Ser Pro Met Lys Lys Leu Ala Phe
                   245                 250                 255
   Arg Gly Ser Val Leu Thr Asp Glu Ser Phe Asn Glu Leu Leu Lys Leu
               260                 265                 270
   Leu Arg Tyr Ile Leu Glu Leu Ser Glu Val Glu Phe Asp Asp Cys Thr
           275                 280                 285
   Leu Asn Gly Leu Gly Asp Phe Asn Pro Ser Glu Ser Asp Val Val Ser
       290                 295                 300
   Glu Leu Gly Lys Val Glu Thr Val Thr Ile Arg Arg Leu His Ile Pro
   305                 310                 315                 320
   Gln Phe Tyr Leu Phe Tyr Asp Leu Ser Thr Val Tyr Ser Leu Leu Glu
                   325                 330                 335
   Lys Val Lys Arg Ile Thr Val Glu Asn Ser Lys Val Phe Leu Val Pro
               340                 345                 350
   Cys Ser Phe Ser Gln His Leu Lys Ser Leu Glu Phe Leu Asp Leu Ser
           355                 360                 365
   Glu Asn Leu Met Val Glu Glu Tyr Leu Lys Asn Ser Ala Cys Lys Gly
       370                 375                 380
   Ala Trp Pro Ser Leu Gln Thr Leu Val Leu Ser Gln Asn His Leu Arg
   385                 390                 395                 400
   Ser Met Gln Lys Thr Gly Glu Ile Leu Leu Thr Leu Lys Asn Leu Thr
                   405                 410                 415
   Ser Leu Asp Ile Ser Arg Asn Thr Phe His Pro Met Pro Asp Ser Cys
               420                 425                 430
   Gln Trp Pro Glu Lys Met Arg Phe Leu Asn Leu Ser Ser Thr Gly Ile
           435                 440                 445
   Arg Val Val Lys Thr Cys Ile Pro Gln Thr Leu Glu Val Leu Asp Val
       450                 455                 460
   Ser Asn Asn Asn Leu Asp Ser Phe Ser Leu Phe Leu Pro Arg Leu Gln
   465                 470                 475                 480
   Glu Leu Tyr Ile Ser Arg Asn Lys Leu Lys Thr Leu Pro Asp Ala Ser
                   485                 490                 495
   Leu Phe Pro Val Leu Leu Val Met Lys Ile Arg Glu Asn Ala Val Ser
               500                 505                 510
   Thr Phe Ser Lys Asp Gln Leu Gly Ser Phe Pro Lys Leu Glu Thr Leu
           515                 520                 525
   Glu Ala Gly Asp Asn His Phe Val Cys Ser Cys Glu Leu Leu Ser Phe
       530                 535                 540
   Thr Met Glu Thr Pro Ala Leu Ala Gln Ile Leu Val Asp Trp Pro Asp
   545                 550                 555                 560
   Ser Tyr Leu Cys Asp Ser Pro Pro Arg Leu His Gly His Arg Leu Gln
                   565                 570                 575
   Asp Ala Arg Pro Ser Val Leu Glu Cys His Gln Ala Ala Leu Val Ser
               580                 585                 590
   Gly Val Cys Cys Ala Leu Leu Leu Leu Ile Leu Leu Val Gly Ala Leu
           595                 600                 605
   Cys His His Phe His Gly Leu Trp Tyr Leu Arg Met Met Trp Ala Trp
       610                 615                 620
   Leu Gln Ala Lys Arg Lys Pro Lys Lys Ala Pro Cys Arg Asp Val Cys
   625 630 635 640
   Tyr Asp Ala Phe Val Ser Tyr Ser Glu Gln Asp Ser His Trp Val Glu
   645 650 655
   Asn Leu Met Val Gln Gln Leu Glu Asn Ser Asp Pro Pro Phe Lys Leu
   660 665 670
   Cys Leu His Lys Arg Asp Phe Val Pro Gly Lys Trp Ile Ile Asp Asn
   675 680 685
   Ile Ile Asp Ser Ile Glu Lys Ser His Lys Thr Val Phe Val Leu Ser
   690 695 700
   Glu Asn Phe Val Arg Ser Glu Trp Cys Lys Tyr Glu Leu Asp Phe Ser
   705 710 715 720
   His Phe Arg Leu Phe Asp Glu Asn Asn Asp Ala Ala Ile Leu Val Leu
   725 730 735
   Leu Glu Pro Ile Glu Arg Lys Ala Ile Pro Gln Arg Phe Cys Lys Leu
   740 745 750
   Arg Lys Ile Met Asn Thr Lys Thr Tyr Leu Glu Trp Pro Leu Asp Glu
   755 760 765
   Gly Gln Gln Glu Val Phe Trp Val Asn Leu Arg Thr Ala Ile Lys Ser
   770 775 780
<210> 3
   <211> 8
   <212> PRT
   <213> Mus musculus
<400> 3
   Gly Phe Thr Phe Thr Thr Tyr Gly
   1 5
<210> 4
   <211> 8
   <212> PRT
   <213> Mus musculus
<400> 4
   Ile Tyr Pro Arg Asp Gly Ser Thr
   1 5
<210> 5
   <211> 11
   <212> PRT
   <213> Mus musculus
<400> 5
   Ala Arg Leu Thr Gly Gly Thr Phe Leu Asp Tyr
   1               5                   10
<210> 6
   <211> 10
   <212> PRT
   <213> Mus musculus
<400> 6
   Glu Ser Val Glu Tyr Tyr Gly Thr Ser Leu
   1               5                   10
<210> 7
   <211> 9
   <212> PRT
   <213> Mus musculus
<400> 7
   Gln Gln Ser Arg Lys Leu Pro Trp Thr
   1               5
<210> 8
   <211> 167
   <212> PRT
   <213> Mus musculus
<400> 8
   Met Ser Ser Pro Gln Ser Leu Lys Thr Leu Ile Leu Thr Met Gly Trp
   1               5                   10                  15
   Ser Trp Ile Phe Leu Phe Leu Leu Ser Gly Thr Ala Gly Val His Ser
               20                  25                  30
   Gln Val Gln Leu Gln Gln Ser Gly Pro Glu Leu Val Asn Pro Gly Ala
           35                  40                  45
   Ser Val Lys Leu Ser Cys Lys Ala Ser Gly Phe Thr Phe Thr Thr Tyr
       50                  55                  60
   Gly Ile Asn Trp Val Lys Gln Gly Pro Gly Gln Gly Leu Glu Trp Ile
   65                  70                  75                  80
   Gly Trp Ile Tyr Pro Arg Asp Gly Ser Thr Asn Phe Asn Glu Asn Phe
                   85                  90                  95
   Lys Asp Lys Ala Ala Leu Thr Val Asp Thr Ser Ser Ser Thr Ala Tyr
               100                 105                 110
   Met Glu Leu His Ser Leu Thr Ser Glu Asp Ser Ala Val Tyr Phe Cys
           115                 120                 125
   Ala Arg Leu Thr Gly Gly Thr Phe Leu Asp Tyr Trp Gly Gln Gly Thr
       130                 135                 140
   Thr Leu Thr Val Ser Ser Ala Lys Thr Thr Pro Pro Ser Val Tyr Pro
   145                 150                 155                 160
   Leu Ala Pro Gly Ser Ala Ala
                   165
<210> 9
   <211> 147
   <212> PRT
   <213> Mus musculus
<400> 9
   Met Glu Ser Asp Thr Leu Leu Leu Trp Val Leu Leu Leu Trp Val Pro
   1               5                   10                  15
   Gly Ser Thr Gly Asp Ile Val Leu Thr Gln Ser Pro Ala Ser Leu Ala
               20                  25                  30
   Val Ser Leu Gly Gln Arg Ala Thr Ile Ser Cys Arg Ala Ser Glu Ser
           35                  40                  45
   Val Glu Tyr Tyr Gly Thr Ser Leu Met Gln Trp Tyr Gln Gln Lys Pro
       50                  55                  60
   Gly Gln Pro Pro Lys Leu Leu Ile Phe Gly Ala Ser Asn Val Glu Ser
   65                  70                  75                  80
   Gly Val Pro Val Arg Phe Ser Gly Ser Gly Ser Gly Thr Asp Phe Ser
                   85                  90                  95
   Leu Asn Ile His Pro Val Glu Glu Asp Asp Ile Val Met Tyr Phe Cys
               100                 105                 110
   Gln Gln Ser Arg Lys Leu Pro Trp Thr Phe Gly Gly Gly Thr Lys Leu
           115                 120                 125
   Glu Ile Lys Arg Ala Asp Ala Ala Pro Thr Val Ser Ile Phe Pro Pro
       130                 135                 140
   Ser Ser Glu
   145
<210> 10
   <211> 111
   <212> PRT
   <213> Homo sapiens
<400> 10
   Asp Ile Val Leu Thr Gln Ser Pro Ala Thr Leu Ser Leu Ser Pro Gly
   1               5                   10                  15
   Glu Arg Ala Thr Leu Ser Cys Arg Ala Ser Glu Ser Val Glu Tyr Tyr
               20                  25                  30
   Gly Thr Ser Leu Met Gln Trp Tyr Gln Gln Lys Pro Gly Gln Pro Pro
           35                  40                  45
   Lys Leu Leu Ile Phe Gly Ala Ser Asn Val Glu Ser Gly Val Pro Asp
       50                  55                  60
   Arg Phe Ser Gly Ser Gly Ser Gly Thr Asp Phe Thr Leu Lys Ile Ser
   65                  70                  75                  80
   Arg Val Glu Ala Glu Asp Val Gly Met Tyr Phe Cys Gln Gln Ser Arg
                   85                  90                  95
   Lys Leu Pro Trp Thr Phe Gly Gly Gly Thr Lys Val Glu Ile Lys
               100                 105                 110
<210> 11
   <211> 118
   <212> PRT
   <213> Homo sapiens
<400> 11
   Gln Val Gln Leu Val Gln Ser Gly Ser Glu Leu Lys Lys Pro Gly Ala
   1               5                   10                  15
   Ser Val Lys Leu Ser Cys Lys Ala Ser Gly Phe Thr Phe Thr Thr Tyr
               20                  25                  30
   Gly Ile Asn Trp Val Arg Gln Ala Pro Gly Gln Gly Leu Glu Trp Ile
           35                  40                  45
   Gly Trp Ile Tyr Pro Arg Asp Gly Ser Thr Asn Phe Asn Glu Asn Phe
       50                  55                  60
   Lys Asp Arg Ala Thr Ile Thr Val Asp Thr Ser Ala Ser Thr Ala Tyr
   65                  70                  75                  80
   Met Glu Leu Ser Ser Leu Arg Ser Glu Asp Thr Ala Val Tyr Phe Cys
                   85                  90                  95
   Ala Arg Leu Thr Gly Gly Thr Phe Leu Asp Tyr Trp Gly Gln Gly Thr
               100                 105                 110
   Thr Val Thr Val Ser Ser
           115
<210> 12
   <211> 218
   <212> PRT
   <213> Homo sapiens
<400> 12
   Asp Ile Val Leu Thr Gln Ser Pro Ala Thr Leu Ser Leu Ser Pro Gly
   1               5                   10                  15
   Glu Arg Ala Thr Leu Ser Cys Arg Ala Ser Glu Ser Val Glu Tyr Tyr
               20                  25                  30
   Gly Thr Ser Leu Met Gln Trp Tyr Gln Gln Lys Pro Gly Gln Pro Pro
           35                  40                  45
   Lys Leu Leu Ile Phe Gly Ala Ser Asn Val Glu Ser Gly Val Pro Asp
       50                  55                  60
   Arg Phe Ser Gly Ser Gly Ser Gly Thr Asp Phe Thr Leu Lys Ile Ser
   65                  70                  75                  80
   Arg Val Glu Ala Glu Asp Val Gly Met Tyr Phe Cys Gln Gln Ser Arg
                   85                  90                  95
   Lys Leu Pro Trp Thr Phe Gly Gly Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 13
   <211> 445
   <212> PRT
   <213> Homo sapiens
<400> 13
   Gln Val Gln Leu Val Gln Ser Gly Ser Glu Leu Lys Lys Pro Gly Ala
   1               5                   10                  15
   Ser Val Lys Leu Ser Cys Lys Ala Ser Gly Phe Thr Phe Thr Thr Tyr
               20                  25                  30
   Gly Ile Asn Trp Val Arg Gln Ala Pro Gly Gln Gly Leu Glu Trp Ile
           35                  40                  45
   Gly Trp Ile Tyr Pro Arg Asp Gly Ser Thr Asn Phe Asn Glu Asn Phe
       50                  55                  60
   Lys Asp Arg Ala Thr Ile Thr Val Asp Thr Ser Ala Ser Thr Ala Tyr
   65                  70                  75                  80
   Met Glu Leu Ser Ser Leu Arg Ser Glu Asp Thr Ala Val Tyr Phe Cys
                   85                  90                  95
   Ala Arg Leu Thr Gly Gly Thr Phe Leu Asp Tyr Trp Gly Gln Gly Thr
               100                 105                 110
   Thr Val Thr Val Ser Ser Ala Ser Thr Lys Gly Pro Ser Val Phe Pro
           115                 120                 125
   Leu Ala Pro Cys Ser Arg Ser Thr Ser Glu Ser Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Lys Thr Tyr Thr Cys Asn Val Asp His Lys Pro Ser
           195                 200                 205
   Asn Thr Lys Val Asp Lys Arg Val Glu Ser Lys Tyr Gly Pro Pro Cys
       210                 215                 220
   Pro Pro Cys Pro Ala Pro Glu Phe Leu Gly Gly Pro Ser Val Phe Leu
   225                 230                 235                 240
   Phe Pro Pro Lys Pro Lys Asp Thr Leu Met Ile Ser Arg Thr Pro Glu
                   245                 250                 255
   Val Thr Cys Val Val Val Asp Val Ser Gln Glu Asp Pro Glu Val Gln
               260                 265                 270
   Phe Asn Trp Tyr Val Asp Gly Val Glu Val His Asn Ala Lys Thr Lys
           275                 280                 285
   Pro Arg Glu Glu Gln Phe Asn Ser Thr Tyr Arg Val Val Ser Val Leu
       290                 295                 300
   Thr Val Leu His Gln Asp Trp Leu Asn Gly Lys Glu Tyr Lys Cys Lys
   305                 310                 315                 320
   Val Ser Asn Lys Gly Leu Pro Ser Ser Ile Glu Lys Thr Ile Ser Lys
                   325                 330                 335
   Ala Lys Gly Gln Pro Arg Glu Pro Gln Val Tyr Thr Leu Pro Pro Ser
               340                 345                 350
   Gln Glu Glu Met Thr Lys Asn Gln Val Ser Leu Thr Cys Leu Val Lys
           355                 360                 365
   Gly Phe Tyr Pro Ser Asp Ile Ala Val Glu Trp Glu Ser Asn Gly Gln
       370                 375                 380
   Pro Glu Asn Asn Tyr Lys Thr Thr Pro Pro Val Leu Asp Ser Asp Gly
   385                 390                 395                 400
   Ser Phe Phe Leu Tyr Ser Arg Leu Thr Val Asp Lys Ser Arg Trp Gln
                   405                 410                 415
   Glu Gly Asn Val Phe Ser Cys Ser Val Met His Glu Ala Leu His Asn
               420                 425                 430
   His Tyr Thr Gln Lys Ser Leu Ser Leu Ser Leu Gly Lys
           435                 440                 445

## Description

### Field of the Invention

The present invention relates to the treatment of Myelodysplastic Syndrome, in subjects who have been shown to be refractory or resistant to treatments with hypomethylating agents. Also described are compositions for use in the treatment of Myelodysplastic Syndrome, in particular subjects who have been shown to be refractory or resistant to treatments such as treatments with hypomethylating agents.

### Background to the Invention

Myelodysplastic Syndrome (MDS) are rare clonal haematopoietic stem cell (HSC) disorders in which immature blood cells in the bone marrow do not mature or become healthy blood cells. They are a complex group of myeloid disorders characterised by ineffective haematopoiesis leading to blood cytopaenias. Their pathophysiology is a multistep process involving cytogenetic or gene changes and widespread gene hypermethylation at advanced stages. Many cases progress to acute myeloid leukaemia (AML).

Problems with blood cell formation result in low red blood cells, low platelets, low white blood cells or a combination thereof leading to the development of different types of MDS. Some types of MDS have an increase in immature blood cells, called blasts, in the bone marrow. The types of MDS are based on specific changes in the blood cells and bone marrow. Some types may develop into acute myeloid leukaemia (AML). A deletion in the long arm of chromosome 5 is associated with dysplastic abnormalities of hematopoietic stem cells (5q-syndrome).

Alterations of innate immune signalling, including overexpression of TLR2, are common in MDS.

Toll-like Receptors (TLRs) form a family of pattern recognition receptors which have a key role in activating the innate immune response. Eleven TLRs have been identified in humans to date. The members of the TLR family are highly conserved, with most mammalian species having between ten to fifteen TLRs. Each TLR recognises specific pathogen-associated molecular signatures. Toll-like Receptor 2 (TLR2, CD282, TLR-2) is activated by peptidoglycan, lipoproteins and lipoteichoic acid. TLR2 is known to dimerise into two functional heterodimers. In particular, TLR2 is known to form a heterodimer with either Toll-like Receptor 1 (TLR1, TLR-1) or Toll-like Receptor 6 (TLR6, TLR-6). It is possible that further heterodimers are formed with Toll-like Receptor 4 (TLR4, TLR-4) and Toll-like Receptor 10 (TLR10, TLR-10). In addition to microbial derived components, TLRs are also known to recognize damage-associated molecular patterns (DAMPs). These are host endogenous molecules released and distributed following stress, tissue damage and cellular disease. WO 2005/028509 discloses a murine IgG1 anti-TLR2 antibody which was derived from hybridoma clone T2.5 (HyCult Biotechnology b.v., Cell Sciences, Canton, USA: catalogue number 1054 - also known as OPN-301). WO2011/003925 describes a humanized version of T2.5 which is designated OPN-305.

Treatments utilised for MDS to date include drug therapy, supportive care and stem cell transplantation. Supportive care may include blood transfusions, medications to increase the making of red blood cells and antibiotics. Drug therapy may include the medication lenalidomide, erythropoiesis stimulating agents, anti-thymocyte globulin and hypomethylating agents such as azacitidine (AZA) and decitabine, among others. Certain people can be treated with chemotherapy followed by a stem cell transplant from a donor. At present, allogeneic haematopoietic stem-cell transplantation is the only treatment that can induce long-term remission in patients with MDS. Such therapy, however, is not applicable for most patients, since the median age at diagnosis exceeds 70 years.

Unfortunately, often these treatments fail. In this case, no further treatments are usually available and the only option is often to simply treat the patient's symptoms. There is thus a need for further treatments of MDS, and in particular further treatments that can be availed of once a previous treatment has failed.

### Summary of the Invention

As described in the examples, the present inventors have shown that in patients identified as being non-responsive to previous anti-MDS treatments, subsequent treatment with a TLR2 antagonist may be efficacious.

The invention is defined in the claims.

The invention provides a Toll-like receptor 2 (TLR2) antagonist for use in the treatment of lower-risk Myelodysplastic Syndrome in a subject who is refractory or resistant to a previous treatment with a hypomethylating agent, wherein the TLR2 antagonist is an antibody or an antigen binding fragment thereof that specifically binds to TLR2, wherein the antibody or an antigen binding fragment thereof specifically binds to a non-continuous epitope comprising amino acid residues His318, Pro320, Arg321, Tyr323, Lys347, Phe349, Leu371, Glu375, Tyr376 and His398 of SEQ ID NO: 1 or His318, Pro320, Gln321, Tyr323, Lys347, Phe349, Leu371, Glu375, Tyr376 and His398 of SEQ ID NO: 2, and wherein said treatment of Myelodysplastic Syndrome is a combination treatment with a therapeutically effective amount of a secondary therapeutic agent which is administered sequentially or simultaneously with said TLR2 antagonist, wherein the secondary therapeutic agent is a hypomethylating agent.

Optionally, the hypomethylating agent is azacitidine or decitabine. Optionally, the hypomethylating agent is azacitidine.

Also described herein is a method for treating Myelodysplastic Syndrome in a subject who is refractory or resistant to a previous treatment, said method comprising the step of:
- administering a therapeutically effective amount of a Toll-like receptor 2 (TLR2) antagonist to said subject.

A previous treatment may involve the administration of a compound selected from the group consisting of a hypomethylating agent (HMA), an erythropoietic stimulating agent (ESA), lenalidomide, an immunotherapeutic agent, a kinase inhibitor, an activin receptor antagonist, a proteasome inhibitor a steroid, an iron chelator, and an anti-neutropenic drug. The hypomethylating agent may be azacitidine or decitabine. Optionally, the hypomethylating agent is azacitidine. A erythropoietic stimulating agent may be erythropoietin (EPO) or darbepoetin (DAR). An immunotherapeutic agent may be anti-thymocyte globulin (ATG), a cyclosporine or checkpoint inhibitor such as a Programmed cell death protein 1 (PD-1) inhibitor, a Programmed death-ligand 1 (PD-L1) inhibitor or a Cytotoxic T-lymphocyte antigen 4 (CTLA-4) inhibitor. A steroid may be prednisolone. An iron chelator may be Exjade. An anti-neutropenic drug may be Neulasta.A previous treatment may be a red blood cell transfusion.

The method described herein may further comprise a step of administering sequentially, separately or simultaneously a therapeutically effective amount of a secondary therapeutic agent suitable for use in the treatment of Myelodysplastic Syndrome. Optionally, the secondary therapeutic agent is a hypomethylating agent. Optionally, the hypomethylating agent is selected from the group consisting of azacitidine and decitabine. Optionally, the combination of treatment with a TLR2 antagonist and a hypomethylating agent demonstrates a synergistic effect in subjects with Myelodysplastic Syndrome.

Also described herein is the use of a composition comprising a Toll-like receptor 2 (TLR2) antagonist in the preparation of a medicament for the treatment of Myelodysplastic Syndrome in a subject who is refractory or resistant to a previous treatment.

Optionally, the medicament is a combined medicament comprising a secondary therapeutic agent. Optionally, the secondary therapeutic agent may be a hypomethylating agent. Optionally, the hypomethylating agent is selected from the group consisting of azacitidine and decitabine. Optionally the hypomethylating agent is azacitidine.

The TLR2 antagonist may be provided in the form of a pharmaceutical composition comprising a TLR2 antagonist, such as a TLR2 antagonistic antibody or antigen binding fragment thereof, at least one pharmaceutically acceptable carrier, diluent, solubilizer, emulsifier, preservative and/or adjuvant and a secondary therapeutic agent, such as a hypomethylating agent.

Also described herein is a kit comprising a TLR2 antagonist and a hypomethylating agent.

Optionally, the hypomethylating agent is azacitidine.

The pharmaceutical composition or the kit may be used for the treatment of Myelodysplastic Syndrome.

Also described herein is a method of treatment of Myelodysplastic Syndrome, said method comprising the simultaneous, sequential or separate administration of a TLR2 antagonist and a hypomethylating agent.

Also described herein is the use of a Toll-like receptor 2 (TLR2) antagonist in the preparation of a medicament for the treatment of Myelodysplastic Syndrome in a subject, wherein said wherein said treatment of Myelodysplastic Syndrome is combination treatment with a therapeutically effective amount of a secondary therapeutic agent which is administered sequentially, separately or simultaneously with said TLR2 antagonist.

Optionally, the subject has lower risk Myelodysplastic Syndrome. The risk of MDS is determined using the International Prognostic Scoring System (IPSS). Optionally, lower risk Myelodysplastic Syndrome includes low risk and intermediate-1 risk subjects. Optionally, the subject has higher risk Myelodysplastic Syndrome. Optionally, the subject is ESA naive. Optionally, the subject is HMA naive. Optionally, the subject is AZA naive. Optionally, lenalidomide is used to as a previous treatment in subjects with a deletion in the long arm of chromosome (del5q subjects). Optionally, the subject may have a low blood cell count. Optionally, the low blood cell count involves red blood cells, white blood cells or platelets. Optionally the subject may be at risk of developing Acute Myeloid Leukaemia.

Optionally, the therapeutically effective amount of the TLR2 antagonist is in the range of 1mg/kg to 20mg/kg. In one embodiment, the therapeutically effective amount of the TLR2 antagonist is 5mg/kg. In one embodiment, the therapeutically effective amount of the TLR2 antagonist is 10mg/kg.

Optionally, the treatment of the present invention reduces the progression of Myelodysplastic Syndrome leading to enhanced survival of, and/or an improved quality of life for, the subject.

As noted above, the disclosure relates to the treatment of Myelodysplastic Syndrome in a subject who is refractory or resistant to a previous MDS treatment. Optionally said subject has previously received said treatment, for example as a sole therapy. In an alternative aspect, said subject has not received said previous treatment but has been determined, for example by a diagnostic test or trial, as being non-responsive to said treatment.

Said previous treatment is treatment or therapy with a hypomethylating agent. A subject with MDS may be considered refractory to a particular treatment where the MDS fails to positively respond to treatment with said agent. Any suitable criteria for assessing a positive response to treatment of MDS may be utilised. For example, a response to a particular treatment may be considered positive if there is an improvement in the IPSS or IPSS-R score of the patient in response to said treatment. Alternatively, a response to a particular treatment may be considered positive if there is an improvement in the hematological profile of the patient in response to said treatment. Optionally, a response to a particular treatment may be considered positive if there is an increase in the hemoglobin level in the patient, for example by at least 0.5g/dL, at least 1g/dL or by at least 2 g/dL, in response to said treatment. In another option, a response to a particular treatment may be considered positive if there is a cytogenetic response to said treatment. In another option, a response to a particular treatment may be considered positive if, in response to said treatment, a subject who was red blood cell transfusion dependent prior to treatment, has reduced dependence, e.g. by at least 25%, at least 50%, or at least 75% on transfusion, or becomes transfusion independent in response to said treatment.

The duration of treatment required with a particular treatment will depend on the specific treatment being used.

A subject with MDS may be considered resistant to a particular treatment where the subject fails to positively respond to treatment with the particular treatment for a time greater than at least 2 months, for example greater than at least at least 4 months or greater than 6 months, or whose MDS progresses within 6 months of completion of treatment with the particular treatment.

Optionally, the previous treatment may have occurred within the previous six months, within the previous twelve months or at any stage of the development of MDS in a subject.

The TLR2 antagonist is an antibody or an antigen binding fragment thereof.

Optionally, the antibody or antigen binding fragment thereof specifically binds to TLR2.

Optionally, the antibody or antigen binding fragment thereof specifically binds to an epitope comprising leucine rich repeat regions 11 to 14 of TLR2.

Optionally, the antibody or an antigen binding fragment thereof specifically binds to a non-continuous epitope comprising amino acid residues His318, Pro320, Arg321, Tyr323, Lys347, Phe349, Leu371, Glu375, Tyr376 and His398 of SEQ ID NO: 1.

Optionally, the antibody or antigen binding fragment thereof specifically binds to a non-continuous epitope comprising amino acid residues His318, Pro320, Gln321, Tyr323, Lys347, Phe349, Leu371, Glu375, Tyr376 and His398 of SEQ ID NO: 2.

Optionally, the antibody or antigen binding fragment comprises a heavy chain variable region comprising a complementarity determining region (CDR) 1 region comprising the amino acid sequence of SEQ ID NO:3, a CDR2 region comprising the amino acid sequence of SEQ ID NO:4 and a CDR3 region comprising the amino acid sequence of SEQ ID NO:5, and/or a light chain variable region comprising a CDR1 region comprising the amino acid sequence of SEQ ID NO:6, a CDR2 region comprising the amino acid sequence Gly-Ala-Ser and a CDR3 region comprising the amino acid sequence of SEQ ID NO:7.

Optionally, the antibody or antigen binding fragment comprises a light chain variable domain comprising an amino acid sequence of SEQ ID NO:10, or a sequence which has at least 90% amino acid sequence identity with SEQ ID NO:10, and/or a heavy chain variable domain comprising an amino acid sequence of SEQ ID NO:11, or a sequence which has at least 90% amino acid sequence identity with SEQ ID NO:1.

Optionally, the antibody or antigen binding fragment specifically binds to TLR2 and antagonises TLR2 function independently of binding of the antibody or antigen binding fragment to CD32.

Optionally, the antibody comprises a heavy chain comprising the amino acid sequence of SEQ ID NO:13, or a sequence which has at least 90% amino acid sequence identity to the amino acid sequence of SEQ ID NO:13, and/or a light chain comprising the amino acid sequence of SEQ ID NO:12, or a sequence which has at least 90% amino acid sequence identity to the amino acid sequence of SEQ ID NO:12, or an antigen binding fragment thereof.

Optionally, the antibody or antigen binding fragment comprises a heavy chain variable region comprising the amino acid sequence of SEQ ID NO:8, or a sequence which has at least 90% amino acid sequence identity to the amino acid sequence of SEQ ID NO:8, and/or a light chain variable region comprising the amino acid sequence of SEQ ID NO:9, or a sequence which has at least 90% amino acid sequence identity to the amino acid sequence of SEQ ID NO:9.

Optionally, the antibody is a humanised version of anti-TLR2 antibody T2.5, or an antigen binding fragment thereof.

Optionally, the antibody or antigen binding fragment suppresses the function of TLR2 irrespective of whether TLR2 forms a heterodimer with TLR1, TLR4, TLR6 or TLR10.

### Brief Description of the Figures

Figure 1 demonstrates the median number of prior therapies for each subject and the duration of prior therapies.
Figure 2 demonstrates the major haematological response in patients 011, 012, and 014.
Figure 3 demonstrates a change in haemoglobin from screening in patients who are HI-E responders (SD: Study day; SW: Study week; CU: Compassionate use; HI-E: Hematologic improvement-Erythroid).
Figure 4A and Figure 4B demonstrate TLR2 blood receptor occupancy after single (Figure 4A) and repeat (Figure 4B) dosing with the TLR2 antibody OPN-305.
Figure 5 demonstrates TLR2 bone marrow receptor occupancy after repeat dosing of the TLR2 antibody OPN-305.
Figure 6A and Figure 6B demonstrate serum concentrations of the TLR2 antibody OPN-305 after single (Figure 6A) and repeat (Figure 6B) dosing.
Figure 7 demonstrates the expressions levels of TLR2 in RNA in subjects who have responded to the treatment of the present invention and subjects who have not responded to the treatment of the present invention.

### Detailed Description

The present inventors have identified that there is a need for treatments of MDS, and in particular treatments that can be availed of once a previous treatment has failed.

The present inventors have shown that a TLR2 antagonist, for example, a TLR2 neutralising antibody, may be used to treat some subjects with MDS shown to be refractory or resistant to a previous treatment.

A previous treatment can be a red blood cell transfusion or can involve the administration of a compound selected from the group consisting of a hypomethylating agent (HMA), an erythropoietic stimulating agent (ESA), lenalidomide, an immunotherapeutic agent, a kinase inhibitor, an activin receptor antagonist, a proteasome inhibitor, a steroid, an iron chelator or an anti-neutropenic drug.

In the invention, the previous treatment involves hypomethylating agent (HMA) therapy, such as with azacitidine (AZA) or decitabine with or without erythropoiesis stimulating agent (ESA).

The hypomethylating agent can be azacitidine or decitabine. The erythropoietic stimulating agent can be erythropoietin (EPO) or darbepoetin (DAR). The immunotherapeutic agent can be anti-thymocyte globulin (ATG), cyclosporine or a checkpoint inhibitor such as a Programmed cell death protein 1 (PD-1) inhibitor, a Programmed death-ligand 1 (PD-L1) inhibitor or a Cytotoxic T-lymphocyte antigen 4 (CTLA-4) inhibitor. The steroid can be prednisolone. The iron chelator can be Exjade. The anti-neutropenic drug can be Neulasta.

The overall response rate in the form of haematological improvement was increased following treatment. The overall response rate was increased by 50% with some subjects achieving transfusion independence. Without wishing to be bound by theory, the inventors predict that treatment with a TLR2 antagonist, for example, a TLR2 neutralising antibody, may improve the haematological profile of subjects with MDS. In particular, the haematological profile of subjects with low or intermediate-1 risk who have undergone a failed therapy is improved. The effect is particularly observed in subjects with overexpression of TLR2, such as subjects who have undergone HMA therapy. Without wishing to be bound by theory, the inventors predict that the overexpression of TLR2 found in MDS subjects is directly related to the response to the TLR2 antagonist observed in the subject. The inventors also predict that the various mutations associated with MDS subjects are directly and individually linked to the response observed in the subject.

One notable advantage of the use of a TLR2 antibody, and in particular OPN-305, in the treatment of MDS is that OPN-305 has a much more desirable safety profile compared to known treatments of MDS. For example, chronic red blood cell transfusions are associated with chronic anemia leading to increased morbidity, especially as a result of cardiac failure, falls, fatigue, and lower quality of life. Treatments with ESA may be associated with an increased risk of stroke, cardio- and thromboembolic events. Treatments with azacitidine may be associated with neutropenia, thrombocytopenia and anemia. Treatments with Lenalidomide are associated with neutropenia and thrombocytopenia and Lenalidomide is a teratogen.

In addition, a reduced frequency of the dose of the TLR2 antagonist may be used compared to known MDS treatments. Based on receptor occupancy data, dosing may potentially be extended beyond current weekly or monthly dosing. Optionally, the frequency of dosing may be extended to every two months.

Furthermore, when the TLR2 antagonist in the form of a TLR2 neutralising antibody was combined with the secondary therapeutic compound, azacitidine, a surprising synergistic effect was observed as compared with treatment with either agent alone. In particular, a combination of azacitidine and TLR2 antagonist was seen to synergistically improve the haematological profile of subjects with MDS and increase survival.

Optionally the TLR2 antagonist is selected from the group consisting of a protein, a peptide, an antibody or an antigen binding fragment thereof, a peptidomimetic, a nucleic acid, a carbohydrate, a lipid and a small molecule.

Optionally the TLR2 antagonist is an antibody or an antigen binding fragment thereof. Typically such an antibody has binding specificity to human TLR2. Typically the antibody has binding specificity to an epitope comprising amino acid residues of the extracellular domain of TLR2. Optionally the antibody binds to the epitope identified above on human TLR2 and/or murine TLR2.

For example, the extracellular domain of the human form of TLR2 comprises 587 amino acid residues, specifically amino acids 1-587 of the 784 amino acid full length human TLR2 sequence shown as SEQ ID NO:1 and defined as Genbank Accession Number AAC 34133 (URL www.ncbi.nlm.nih.gov). An anti-TLR2 antibody may bind to the ligand binding region of TLR2. Optionally the TLR2 antagonist inhibits or prevents dimerization of TLR2 with Toll-like Receptor 1 (TLR1) or Toll-like Receptor 6 (TLR6) by covering the interaction surface between TLR2 and TLR1 or TLR6. Typically the binding region/interaction surface is located within leucine-rich repeat (LRR) regions 11 to 14 of TLR2. Optionally the TLR2 antagonist has binding specificity to an epitope comprising, consisting of or consisting essentially of LRR regions 11 to 14 of TLR2, or a sequence which has at least 85%, 90% or 95% sequence identity thereto.

Optionally a TLR2 antibody binds to a non-continuous epitope comprising, consisting of or consisting essentially of amino acid residues His318 (H, histidine), Pro320 (P, proline), Arg321 (R, arginine) or Gln321 (Q, glutamine), Tyr323 (Y, tyrosine), Lys347 (K, lysine), Phe349 (F, phenylalanine), Leu371 (L, leucine), Glu375 (E, glutamic acid), Tyr376 (Y, tyrosine), and His398 (H, histidine) of SEQ ID NO: 1 or SEQ ID NO:2.

Optionally a TLR2 antibody binds to a non-continuous epitope comprising, consisting of or consisting essentially of amino acid residues His318 (H, histidine), Pro320 (P, proline), Arg321 (R, arginine), Tyr323 (Y, tyrosine), Lys347 (K, lysine), Phe349 (F, phenylalanine), Leu371 (L, leucine), Glu375 (E, glutamic acid), Tyr376 (Y, tyrosine), and His398 (H, histidine) of SEQ ID NO: 1.

Optionally the TLR2 antibody binds to a non-continuous epitope comprising, consisting of or consisting essentially of amino acid residues His318 (H, histidine), Pro320 (P, proline), Gln321 (Q, glutamine), Tyr323 (Y, tyrosine), Lys347 (K, lysine), Phe349 (F, phenylalanine), Leu371 (L, leucine), Glu375 (E, glutamic acid), Tyr376 (Y, tyrosine), and His398 (H, histidine) of murine TLR2 (SEQ ID NO: 2). SEQ ID NO:2 shows the amino acid murine TLR2 sequence defined as Genbank Accession Number NP_036035 (Mus musculus).

The above identified epitope in humans or mice is a functional epitope for receptor dimerization or inhibition thereof. Typically the epitope may be bound by the TLR2 antagonistic antibodies T2.5 or OPN-305. Binding of the identified epitope by a binding member, such as an antibody, results in antagonism of TLR2 biological function, in particular activation and signalling. In particular, binding by the binding member serves to inhibit activation of the TLR2 receptor, irrespective of whether a TLR2 heterodimer is formed with another TLR, such as TLR1, TLR6, TLR4 or TLR10. Furthermore, antibodies binding to this epitope have been shown to cross-react with TLR2 from human, pig and monkey, indicating this to be a critical epitope in a highly conserved region of TLR2.

Optionally the antibody is selected from the group consisting of a human antibody, a humanised antibody, a chimeric antibody, a monoclonal antibody, a polyclonal antibody, a synthetic antibody, a camelid antibody, a shark antibody and an *in-vitro* antibody. Optionally an antigen binding fragment may be used. The antigen binding fragment may be derived from any of the aforementioned antibodies. Optionally the antigen binding fragment is selected from the group consisting of a Fab fragment, a scFv fragment, a Fv fragment and a dAb fragment. Optionally the antibody comprises two complete heavy chains and two complete light chains, or an antigen binding fragment thereof. Optionally the antibody is of the isotype IgG, IgA, IgE or IgM, or an antigen binding fragment thereof. Optionally where the antibody is of the isotype IgG, the antibody may be of the subtype IgG1, IgG2 or IgG3, or an antigen binding fragment thereof. Optionally the antibody is of the subtype IgG4, or an antigen binding fragment thereof. The antibody or antigen binding fragment may bind to an inhibitory epitope present on TLR2 with a dissociation constant (Kd) of from about 10⁻⁷M to about 10⁻¹¹M. More particularly the antibody or antigen binding fragment thereof may bind to mammalian (e.g. human or murine) TLR2 with a K_{D} of 4 x 10⁻⁸ M or less. Even more particularly the antibody or antigen binding fragment thereof may bind to human TLR2 with a K_{D} of 3 x 10⁻⁸ M or less. Optionally the antibody is an isolated antibody or an antigen binding fragment thereof.

Any suitable TLR2 antagonist antibody may be used. Optionally the antibody or antigen binding fragment comprises a heavy chain variable region comprising the heavy chain variable region complementarity regions of the murine IgG1 anti-TLR2 antibody derived from hybridoma clone T2.5 (HyCult Biotechnology b.v., Cell Sciences, Canton, USA: catalogue number 1054) and/or a light chain variable region comprising the light chain variable region complementarity regions of the murine IgG1 anti-TLR2 antibody derived from hybridoma clone T2.5 (HyCult Biotechnology b.v., Cell Sciences, Canton, USA: catalogue number 1054). Optionally the antibody or antigen binding fragment comprises a heavy chain variable region comprising a complementarity determining region 1 (CDR1) region comprising the amino acid sequence Gly-Phe-Thr-Phe-Thr-Thr-Tyr-Gly (SEQ ID NO:3), a CDR2 region comprising the amino acid sequence Ile-Tyr-Pro-Arg-Asp-Gly-Ser-Thr (SEQ ID NO:4) and a CDR3 region comprising the amino acid sequence Ala-Arg-Leu-Thr-Gly-Gly-Thr-Phe-Leu-Asp-Tyr (SEQ ID NO:5), and/or a light chain variable region comprising a CDR1 region comprising the amino acid sequence Glu-Ser-Val-Glu-Tyr-Tyr-Gly-Thr-Ser-Leu (SEQ ID NO:6), a CDR2 region comprising the amino acid sequence Gly-Ala-Ser and a CDR3 region comprising the amino acid sequence Gln-Gln-Ser-Arg-Lys-Leu-Pro-Trp-Thr (SEQ ID NO:7).

Optionally the antibody or antigen binding fragment comprises a heavy chain variable region of the murine IgG1 anti-TLR2 antibody derived from hybridoma clone T2.5 (HyCult Biotechnology b.v., Cell Sciences, Canton, USA: catalogue number 1054) and/or a light chain variable region of the murine IgG1 anti-TLR2 antibody derived from hybridoma clone T2.5 (HyCult Biotechnology b.v., Cell Sciences, Canton, USA: catalogue number 1054). Optionally the heavy chain variable region comprises or consists of the amino acid sequence as depicted in SEQ ID NO:8, or a sequence which has at least 85%, 90%, 95%, 96%, 97%, 98% or 99% amino acid sequence identity over a length of at least 20, and up to all, amino acids of the amino acid sequence of SEQ ID NO:8, and/or the light chain variable region comprises or consists of the amino acid sequence as depicted in SEQ ID NO:9, or a sequence which has at least 85%, 90%, 95%, 96%, 97%, 98% or 99% amino acid sequence identity over a length of at least 20, and up to all, amino acids of the amino acid sequence of SEQ ID NO:9.

Optionally the antibody or antigen binding fragment comprises a heavy chain variable region of a humanised version of the murine IgG1 anti-TLR2 antibody derived from hybridoma clone T2.5 (HyCult Biotechnology b.v., Cell Sciences, Canton, USA: catalogue number 1054) and/or a light chain variable region of a humanised version the murine IgG1 anti-TLR2 antibody derived from hybridoma clone T2.5 (HyCult Biotechnology b.v., Cell Sciences, Canton, USA: catalogue number 1054). Optionally the antibody or antigen binding fragment comprises a heavy chain variable region of the OPN-305 antibody as described in WO2011/003925 and/or a light chain variable region of the OPN-305 antibody as described in WO2011/003925. Optionally the antibody or antigen binding fragment comprises a light chain variable domain comprising or consisting of an amino acid sequence of SEQ ID NO:10, or a sequence which has at least 85%, 90%, 95%, 96%, 97%, 98% or 99% amino acid sequence identity over a length of at least 20, and up to all, amino acids of the amino acid sequence of SEQ ID NO:10, and/or a heavy chain variable domain comprising or consisting of an amino acid sequence of SEQ ID NO:11, or a sequence which has at least 85%, 90%, 95%, 96%, 97%, 98% or 99% amino acid sequence identity over a length of at least 20, and up to all, amino acids of the amino acid sequence of SEQ ID NO:11. Typically, the antibody or antigen binding fragment specifically binds to TLR2 and does not bind to CD32. Optionally, the antibody or antigen binding fragment thereof mediates TLR2 antagonism independently of binding of the antibody or antigen binding fragment thereof to TLR2.

Optionally the antibody or antigen binding fragment comprises a heavy chain of a humanised version of the murine IgG1 anti-TLR2 antibody derived from hybridoma clone T2.5 (HyCult Biotechnology b.v., Cell Sciences, Canton, USA: catalogue number 1054) and/or a light chain of a humanised version the murine IgG1 anti-TLR2 antibody derived from hybridoma clone T2.5 (HyCult Biotechnology b.v., Cell Sciences, Canton, USA: catalogue number 1054), or an antigen binding fragment thereof. Optionally the antibody or antigen binding fragment comprises a heavy chain of the OPN-305 antibody as described in WO2011/003925 and/or a light chain of the OPN-305 antibody as described in WO2011/003925. Optionally the light chain comprises or consists of the amino acid sequence of SEQ ID NO:12, or a sequence which has at least 85%, 90%, 95%, 96%, 97%, 98% or 99% amino acid sequence identity over a length of at least 20, and up to all, amino acids of the amino acid sequence of SEQ ID NO:12, and/or the heavy chain comprises the amino acid sequence of SEQ ID NO:13, or a sequence which has at least 85%, 90%, 95%, 96%, 97%, 98% or 99% amino acid sequence identity over a length of at least 20, and up to all, amino acids of the amino acid sequence of SEQ ID NO:13. Optionally, the antibody or antigen binding fragment is a fully humanised antibody. Optionally the variable domain of the heavy chain is joined to a constant domain derived from an antibody of the subclass immunoglobulin G, isotype 4. Optionally amino acid residue 241 of a hinge region of the heavy chain is substituted from a serine residue to a proline residue. Optionally the antibody or antigen binding fragment binds to mammalian Toll-like Receptor 2 with a K_{D} of 1x10⁻⁸ M or less. Optionally the antibody or antigen binding fragment binds to mammalian (e.g. human or murine) TLR2 with a K_{D} of 4 x 10⁻⁸ M or less. Optionally the antibody or antigen binding fragment binds to human TLR2 with a K_{D} of 3 x 10⁻⁸ M or less.

Optionally the antibody or antigen binding fragment is derived from hybridoma clone T2.5 (HyCult Biotechnology b.v., Cell Sciences, Canton, USA: catalogue number 1054), or is a humanised version thereof. Optionally the antibody is a murine IgG1 anti-TLR2 antibody derived from hybridoma clone T2.5 (HyCult Biotechnology b.v., Cell Sciences, Canton, USA: catalogue number 1054), or a humanised version or antigen binding fragment thereof. Optionally the humanised version thereof is the OPN-305 antibody, as described in WO2011/003925.

A TLR2 antagonist may comprise a nucleic acid encoding an antibody or antigen binding fragment as described above, or a vector comprising said nucleic acid.

A TLR2 antagonist may be an inhibitory nucleic acid which inhibits the expression of at least one nucleic acid which encodes TLR2 protein. A TLR2 antagonist is selected from the group consisting of anti-sense oligonucleotides, triple helix molecules, anti-sense DNA, anti-sense RNA, ribozyme, iRNA, miRNA, siRNA and shRNA. Accordingly, also described herein is the administration to the subject of a therapeutically effective amount of an inhibitory nucleic acid, such as an RNAi (RNA interference) agent, for example an interfering ribonucleic acid (such as siRNA or shRNA) or a transcription template thereof, such as a DNA encoding an shRNA in order to block the expression of the TLR2 protein. Optionally the inhibitory nucleic acid may be an antisense RNA molecule. Antisense causes suppression of gene expression and involves single stranded RNA fragments which physically bind to mRNA, thus blocking mRNA translation. Techniques for the preparation of inhibitory nucleic acids are known to persons skilled in the art.

The inventors have further identified that suppression of the function of TLR2 can be achieved by means of reducing the amount of ligand which is available to bind to and activate membrane bound TLR2. A reduction in the amount of ligand which is available to bind membrane bound TLR2 results in a downregulation of TLR2-mediated signalling. Accordingly, Optionally the TLR2 antagonist is a soluble form of recombinant TLR2 (sTLR2), or a functional fragment thereof. The soluble form of TLR2 competes with the membrane bound form of TLR2 for TLR2 specific binding ligands. This competitive binding results in the soluble form of TLR2 effectively "mopping up" available TLR2 ligand with an associated reduction in the binding and activation of membrane bound TLR2.

Optionally the soluble form of TLR2 is prepared by a recombinant technique. A soluble form of TLR2 typically comprises the extracellular domain of TLR2 only, and hence the intracellular and transmembrane domains of TLR2 as defined in Genbank Accession Number AAC 34133 (SEQ ID NO:1) are absent. Optionally, the soluble form of TLR2 comprises amino acids 1 to 587 of SEQ ID NO:1. The soluble TLR2 sequence may be modified by means of the addition, deletion or substitution of one or more amino acid residues. Accordingly, optionally the soluble form of TLR2 is derived from a truncated form of the full length membrane bound TLR2 amino acid sequence or, in addition to a deletion and/or substitution of the amino acids residues relating to the intracellular and/or transmembrane domains in the membrane bound form of TLR2, a deletion and/or substitution may further be made to the amino acid residues of the extracellular domain. Any such truncation or deletion and/or substitution of the amino acid residues of the extracellular domain of the TLR2 may be made so long as the modified form of TLR2 is soluble and capable of binding a ligand which can bind to at least one epitope present on the membrane bound form of TLR2.

Optionally the TLR2 antagonist, such as the soluble form of TLR2, is targeted to haematopoietic cells or TLR2 expressed on haematopoietic cells. Targeting may be by any suitable means known to the person skilled in the art, such as localised delivery, the use of a delivery vector or a targeting means, such as an antibody which has binding specificity for a cell surface target expressed on haematopoietic cells. The targeting of the TLR2 antagonist in this way is advantageous as systemic administration of the TLR2 antagonist may result in global immunosuppression of the TLR2 receptor and accordingly TLR2 mediated signalling which may be undesirable in some instances. Targeting of the TLR2 antagonist, such as the soluble form TLR2, may be provided through the formation of a fusion protein, wherein said fusion protein is comprised of a TLR2 antagonist (e.g. a soluble portion of the TLR2 receptor, typically the extracellular domain or a portion thereof) conjoined to a secondary peptide, typically the Fc receptor binding protein derived from the heavy chain of an immunoglobulin, typically a human immunoglobulin. The Fc domain has been extensively used to prolong the circulatory half-life of therapeutic proteins.

Optionally the TLR2 antagonist binds to the epitope present on the extracellular domain of TLR2 with an affinity constant (Ka) of at least 10⁻⁶M.

Also described herein is a screening method for the identification of compounds for use in treatment of Myelodysplastic Syndrome in a subject who is refractory or resistant to treatment with a previous treatment, the method comprising the steps of:
(a) providing candidate compounds, e.g. antibodies having binding specificity for TLR2;
(b) contacting the candidate compounds with TLR2; and
(c) identifying compounds which bind to TLR2 within the region of amino acid residues His318 (H, histidine), Pro320 (P, proline), Arg321 (R, arginine) or Gln321 (Q, glutamine), Tyr323 (Y, tyrosine), Lys347 (K, lysine), Phe349 (F, phenylalanine), Leu371 (L, leucine), Glu375 (E, glutamic acid), Tyr376 (Y, tyrosine), and His398 (H, histidine) of SEQ ID NO: 1 or SEQ ID NO:2;
wherein binding in this region is indicative of utility of the compound in the treatment of Myelodysplastic Syndrome in a subject who has received a previous treatment.

Also described is a screening method for the identification of compounds for use in treatment of Myelodysplastic Syndrome in a subject who is refractory or resistant to a previous treatment, the method comprising the steps of:
(a) providing candidate compounds which are antagonists of TLR2 function, e.g. antibodies having binding specificity for TLR2 or antigen binding fragments thereof;
(b) contacting the candidate compounds with TLR2; and
(c) identifying compounds which bind to TLR2 within the region of amino acid residues His318 (H, histidine), Pro320 (P, proline), Arg321 (R, arginine) or Gln321 (Q, glutamine), Tyr323 (Y, tyrosine), Lys347 (K, lysine), Phe349 (F, phenylalanine), Leu371 (L, leucine), Glu375 (E, glutamic acid), Tyr376 (Y, tyrosine), and His398 (H, histidine) of SEQ ID NO: 1 or SEQ ID NO:2;
wherein binding in this region is indicative of utility of the compound in the treatment of Myelodysplastic Syndrome in a subject who has received a previous treatment.

### Definitions

As herein defined, "Toll-like Receptor 2" may be also referred to as TLR2, TLR-2 or CD282. Typically, the TLR2 is human TLR2. Alternatively, the TLR2 is murine TLR2. In further embodiments, the TLR2 is a homologue or orthologue of human TLR2 which is derived from any mammal other than a human or mouse, for example, a cow or rat. Optionally the TLR2 antagonist is cross-reactive in that it mediates the suppression of TLR2 function in TLR2 derived from different species.

As herein defined, an agent is a "TLR2 antagonist" if the agent suppresses or blocks the activation or function of TLR2. The agent may inhibit or block binding of a ligand or binding compound to TLR2. This inhibition of TLR2 ligand binding may be achieved by a number of means, for example, through binding to the extracellular domain of TLR2 and partially or fully blocking the TLR2 ligand binding site, or by binding at a site other than the known TLR2 ligand binding site and inducing a conformational change upon binding which results in the TLR2 ligand binding site being altered in a manner which prevents TLR2 ligand binding or TLR2 activation. Further, the agent may inhibit or suppress intracellular signalling mediated by TLR2 following ligand binding and/or TLR2 activation.

Intracellular signalling mediated following TLR2 activation and signalling results in the activation of transcription factors and the expression of genes which mediate a pro-inflammatory immune response. The agent may suppress or block TLR2 protein or gene expression, for example, by inhibiting the expression of a gene encoding a TLR2 protein.

As herein defined, the terms "blocks" and "blocking" when used in relation to TLR2 gene expression mean silencing the expression of at least one gene which results in the expression of the TLR2 protein. Gene silencing is the switching off of the expression of a gene by a mechanism other than genetic modification. Gene silencing can be mediated at the transcriptional level or at the post-transcriptional level. Transcriptional gene silencing can result in a gene being inaccessible to transcriptional machinery, and can be mediated, for example, by means of histone modifications. Post-transcriptional gene silencing results from the mRNA of a gene being destroyed, thus preventing an active gene product, such as a protein, in the present case the TLR2 protein.

The term "specifically binds" or "binding specificity" refers to the ability of a TLR2 binding compound to bind to a target epitope present on TLR2 with a greater affinity than it binds to a non-target epitope. Optionally specific binding refers to binding to a particular target epitope which is present on TLR2 with an affinity which is at least 10, 50, 100, 250, 500, or 1000 times greater than the affinity for a non-target epitope. Binding affinity may be determined by an affinity ELISA assay, a BIAcore assay, a kinetic method or by an equilibrium/solution method.

As herein defined, an "epitope" refers to a plurality of amino acid residues which are capable of being recognised by, and bound to by, a binding compound, such as a ligand, small molecule or antibody. Epitopes are generally comprised of chemically active surface groups and have specific three-dimensional structural characteristics, as well as specific charge characteristics which contribute to the three-dimensional structure of the epitope. The TLR2 antagonist antagonises the functional activity of TLR2 and as such binds to an epitope known as an inhibiting epitope or an inhibitory epitope. An "inhibiting" or "inhibitory" epitope means an epitope present on TLR2 that, when bound by a binding compound such as a small molecule or an antibody, results in the loss of biological activity of TLR2, for example due to the binding compound preventing the binding of TLR2 by a TLR2 agonist. The epitope that is present on TLR2, and which is bound by the binding compounds in order to antagonise TLR2 function, may comprise five or more amino acid residues.

The TLR2 antagonist of the invention binds to a non-contiguous epitope. A "non-contiguous epitope" is an epitope that is comprised of a series of amino acid residues that are non-linear in alignment, such that the residues are spaced or grouped in a non-continuous manner along the length of a polypeptide sequence. The non-contiguous epitope described herein is a discontinuous scattered epitope wherein the residues which contribute to the epitope are provided in three or more groups of linear amino acid sequences arranged along the length of the TLR2 polypeptide.

As used herein, the term "subject" refers to an animal, preferably a mammal and in particular a human.

The term "consisting essentially of" as used herein means that the invention necessarily includes the listed items and is open to including unlisted items that do not materially affect the basic and novel properties of the invention.

The nomenclature used to describe the polypeptide constituents utilised Optionally of the present invention follows the conventional practice wherein the amino group (N) is presented to the left and the carboxyl group to the right of each amino acid residue.

The expression "amino acid" as used herein is intended to include both natural and synthetic amino acids, and both D and L amino acids. A synthetic amino acid also encompasses chemically modified amino acids, including, but not limited to, salts, and amino acid derivatives such as amides. Amino acids can be modified by methylation, amidation, acetylation or substitution with other chemical groups which can change the circulating half life without adversely affecting their biological activity.

The terms "peptide", "polypeptide" and "protein" are used herein interchangeably to describe a series of at least two amino acids covalently linked by peptide bonds or modified peptide bonds such as isosteres. No limitation is placed on the maximum number of amino acids which may comprise a peptide or protein. Furthermore, the term polypeptide extends to fragments, analogues and derivatives of a peptide, wherein said fragment, analogue or derivative retains the same biological functional activity as the peptide from which the fragment, derivative or analogue is derived.

Furthermore the term "fusion protein" as used herein can also be taken to mean a fusion polypeptide, fusion peptide or the like, or may also be referred to as an immunoconjugate. The term "fusion protein" refers to a molecule in which two or more subunit molecules, typically polypeptides, are covalently or non-covalently linked.

As used herein, the term "therapeutically effective amount" means an amount which is sufficient to show benefit to the subject to whom the composition or TLR2 antagonist is administered, e.g. by reducing the severity of at least one symptom of Myelodysplastic Syndrome e.g. by reducing the concentration of leukemic blast cells in the bone marrow, by increasing blood cell count, by suppressing a T regulatory cell response and/or by promoting a Th1 response..

As used herein, the term "treatment" and associated terms such as "treat" and "treating" means the reduction of the progression or severity of Myelodysplastic Syndrome or at least one symptom thereof, e.g. by reducing the concentration of leukemic blast cells in the bone marrow, by increasing blood cell count, by suppressing a T regulatory cell response and/or by promoting a Th1 response. The term "treatment" refers to any regimen that can benefit a subject. The treatment may be in respect of an existing Myelodysplastic Syndrome. Treatment may include curative, alleviative or prophylactic effects. References herein to "therapeutic" and "prophylactic" treatments are to be considered in their broadest context. The term "therapeutic" does not necessarily imply that the subject is treated until total recovery. Similarly, "prophylactic" does not necessarily mean that the subject will not eventually contract a disease condition.

Unless otherwise defined, all technical and scientific terms used herein have the meaning commonly understood by a person who is skilled in the art in the field of the present invention.

Throughout the specification, unless the context demands otherwise, the terms "comprise" or "include", or variations such as "comprises" or "comprising", "includes" or "including" will be understood to imply the inclusion of a stated integer or group of integers, but not the exclusion of any other integer or group of integers.

As used herein, terms such as "a", "an" and "the" include singular and plural referents unless the context clearly demands otherwise. Thus, for example, reference to "an active agent" or "a pharmacologically active agent" includes a single active agent as well as two or more different active agents in combination, while references to "a carrier" includes mixtures of two or more carriers as well as a single carrier, and the like.

### International Prognostic Scoring System (IPSS)

The current standard way to define risk in MDS is by using the International Prognostic Scoring System (IPSS). This system uses three criteria to assess whether MDS is classified as lower- or higher-risk. The criteria are as follows:
(i) The percentage of leukemic blast cells, or immature white blood cells, in the marrow
(ii) The type of chromosomal changes, if any, in the marrow cells (cytogenetics)
(iii) The presence of one or more low blood cell counts (cytopenias).

A score is assigned based on these three factors. The score will fall into one of four categories. These include the low, intermediate-1, intermediate-2, and high risk categories. The low and intermediate-1 risk categories are considered lower-risk MDS, and the intermediate-2 and high-risk categories are considered higher-risk MDS.

### Revised International Prognostic Scoring System (IPSS-R)

The revised IPSS, known as the "IPSS-R," covers the same disease factors as the IPSS, but the factors are identified in a more detailed way. The IPSS-R shows five disease factors (blasts, cytogenetics, hemoglobin, absolute neutrophil count, and platelet count). A patient's IPSS-R risk category is determined by totalling the individual IPSS-R scores for the designated values within the five disease factors. That number matches the patient to one of the following five IPSS-R risk categories: Very low, Low, Intermediate, High and Very High.

### Antibodies

A TLR2 antagonist may be an antibody or an antigen binding fragment thereof. An "antibody" is an immunoglobulin, whether natural or partly or wholly synthetically produced. The term also covers any polypeptide, protein or peptide having a binding domain that is, or is homologous to, an antibody binding domain. These can be derived from natural sources, or they may be partly or wholly synthetically produced. Examples of antibodies are the immunoglobulin isotypes and their isotypic subclasses and fragments which comprise an antigen binding domain such as Fab, scFv, Fv, dAb or Fd, and a bi-specific antibody.

Optionally the antibody may be a camelid antibody, in particular a camelid heavy chain antibody. Further, the antibody fragment may be a domain antibody or a nanobody derived from a camelid heavy chain antibody. Optionally the antibody may be a shark antibody or a shark derived antibody.

Optionally the antibody is an "isolated antibody", this meaning that the antibody is (1) free of at least some proteins with which it would normally be found, (2) is essentially free of other proteins from the same source, e.g., from the same species, (3) is expressed by a cell from a different species, or (4) does not occur in nature.

As antibodies can be modified in a number of ways, the term "antibody" should be construed as covering any binding member or substance having a binding domain with the required specificity. The antibody of the invention may be a monoclonal antibody, or a fragment, derivative, functional equivalent or homologue thereof. The term includes any polypeptide comprising an immunoglobulin binding domain, whether natural or wholly or partially synthetic. Chimeric molecules comprising an immunoglobulin binding domain, or equivalent, fused to another polypeptide are therefore included.

The constant region of the antibody may be of any suitable immunoglobulin subtype. However, it is preferred that the antibody subtype is IgG1. In alternative embodiments the subtype of the antibody may be of the class IgA, IgM, IgD and IgE where a human immunoglobulin molecule is used. Such an antibody may further belong to any subclass e.g. IgG1, IgG2a, IgG2b, IgG3 and IgG4.

Fragments of a whole antibody can perform the function of antigen binding. Examples of such binding fragments are a Fab fragment comprising of the VL, VH, CL and CH1 antibody domains; an Fv fragment consisting of the VL and VH domains of a single antibody; a F(ab')2 fragment; a bivalent fragment comprising two linked Fab fragments; a single chain Fv molecule (scFv), wherein a VH domain and a VL domain are linked by a peptide linker which allows the two domains to associate to form an antigen binding site; and a bi-specific antibody, which may be multivalent or multispecific fragments constructed by gene fusion.

A fragment of an antibody or of a polypeptide for use in the present invention, for example, a fragment of a TLR2 specific antibody, generally means a stretch of amino acid residues of at least 5 to 7 contiguous amino acids, often at least about 7 to 9 contiguous amino acids, typically at least about 9 to 13 contiguous amino acids, more preferably at least about 20 to 30 or more contiguous amino acids and most preferably at least about 30 to 40 or more consecutive amino acids.

A "derivative" of such an antibody or polypeptide, or of a fragment of a TLR2 specific antibody means an antibody or polypeptide modified by varying the amino acid sequence of the protein, e.g. by manipulation of the nucleic acid encoding the protein or by altering the protein itself. Such derivatives of the natural amino acid sequence may involve insertion, addition, deletion and/or substitution of one or more amino acids, preferably while providing a peptide having TLR2 binding activity. Preferably such derivatives involve the insertion, addition, deletion and/or substitution of 25 or fewer amino acids, more preferably of 15 or fewer, even more preferably of 10 or fewer, more preferably still of 4 or fewer and most preferably of 1 or 2 amino acids only.

Humanized antibodies may be utilised in the present invention. A humanised antibody may be a modified antibody having the hypervariable region of a TLR2 specific antibody and the constant region of a human antibody. Thus the binding member may comprise a human constant region. The variable region other than the hypervariable region may also be derived from the variable region of a human antibody and/or may also be derived from a TLR2 specific antibody. In other cases, the entire variable region may be derived from a murine monoclonal TLR2 specific antibody and the antibody is said to be chimerised.

It is possible to take monoclonal and other antibodies and use techniques of recombinant DNA technology to produce other antibodies or chimeric molecules which retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. A hybridoma or other cell producing an antibody may be subject to genetic mutation or other changes, which may or may not alter the binding specificity of antibodies produced.

Optionally the therapeutically effective amount comprises the antibody in a range 1 mg/kg to 20 mg/kg, such as, 1 mg/kg to 10 mg/kg, 5 mg/kg to 10 mg/kg or 10 mg/kg to 20 mg/kg. Optionally the therapeutically effective amount comprises the antibody in an amount of about 5 mg/kg. Optionally the therapeutically effective amount comprises the antibody in an amount of about 10 mg/kg.

The present invention extends to the use of sequences having at least 80%, 85%, 90%, 95%, 97%, 98% or 99% amino acid sequence identity to the sequences of the TLR2 antibodies described herein. Such sequences or polypeptides may comprise a sequence which is substantially homologous to a polypeptide having the amino acid sequence of a TLR2 antagonist described herein, but may have a different amino acid sequence because of one or more deletions, insertions, or substitutions. The substantially homologous polypeptide may include 1, 2 or more amino acid alterations. Alternatively, or in addition, the substantially homologous polypeptide may consist of a truncated version of a TLR2 antagonist described herein which has been truncated by 1, 2 or more amino acids. Optionally sequence identity is over a length of at least 20, 25, 30, 35 or 40 amino acids of the amino acid sequence in question. Optionally sequence identity is over the complete length of the amino acid sequence in question (e.g. any one of SEQ ID NO:8, 9, 10, 11, 12 and/or 13).

A given amino acid may be replaced, for example, by a residue having similar physiochemical characteristics. Examples of such conservative substitutions include substitution of one aliphatic residue for another, such as Ile, Val, Leu, or Ala for one another; substitutions of one polar residue for another, such as between Lys and Arg, Glu and Asp, or Gln and Asn; or substitutions of one aromatic residue for another, such as Phe, Trp, or Tyr for one another. Other conservative substitutions, e.g., involving substitutions of entire regions having similar hydrophobicity characteristics, are well known.

Similarly, the nucleic acids for use herein may be variants that differ from a native DNA sequence because of one or more deletions, insertions or substitutions, but that encode a biologically active polypeptide.

As used herein, percentage amino acid sequence identity may be determined, using any method known to the person skilled in the art, for example, by comparing sequence information using the GAP computer program, version 6.0 described by Devereux et al. (Nucl. Acids Res. 12:387, 1984) and available from the University of Wisconsin Genetics Computer Group (UWGCG).

### Production of Antibodies

Antibodies for use in the present invention may be provided by a number of techniques. For example, a combinatorial screening technique such as a phage display-based biopanning assay may be used to in order to identify amino acid sequences which have binding specificity to the binding epitopes described above. Such phage display biopanning techniques involve the use of phage display libraries, which are utilised in methods which identify suitable epitope binding ligands in a procedure which mimics immune selection, through the display of antibody binding fragments on the surface of filamentous bacteria. Phage with specific binding activity are selected. The selected phage can thereafter be used in the production of chimeric, CDR-grafted, humanised or human antibodies.

In further embodiments the antibody is a monoclonal antibody, which may be produced using any suitable method which produces antibody molecules by continuous cell lines in culture. Chimeric antibodies or CDR-grafted antibodies are further provided for use in the present invention. In certain embodiment, antibodies for use in the invention may be produced by the expression of recombinant DNA in host cell.

Optionally the monoclonal antibodies may be human antibodies, produced using transgenic animals, for example, transgenic mice, which have been genetically modified to delete or suppress the expression of endogenous murine immunoglobulin genes, with loci encoding for human heavy and light chains being expressed in preference, this resulting in the production of fully human antibodies.

Optionally the TLR2 antagonist is a binding fragment which is derived from an antibody, for example, an antibody binding fragment, such as a Fab, F(ab')2, Fv or a single chain Fv (scFV).

Optionally the TLR2 antagonist comprises a polyclonal antibody, a chimeric antibody, a synthesized or synthetic antibody, a fusion protein or fragment thereof, a natural or synthetic chemical compound or a peptidomimetic.

Antibodies or antigen fragments for use in the present invention may also be generated wholly or partly by chemical synthesis. The antibodies can be readily prepared according to well-established, standard liquid or, preferably, solid-phase peptide synthesis methods, general descriptions of which are broadly available and are well known by the person skilled in the art. Further, they may be prepared in solution, by the liquid phase method or by any combination of solid-phase, liquid phase and solution chemistry.

Another convenient way of producing antibodies or antibody fragments suitable for use in the present invention is to express nucleic acid encoding them by use of nucleic acid in an expression system.

### Nucleic Acid

Nucleic acid may comprise DNA or RNA and may be wholly or partially synthetic. Optionally, nucleic acid codes for antibodies or antibody fragments of the invention as defined above. The skilled person will be able to determine substitutions, deletions and/or additions to such nucleic acids which will provide an antibody or antibody fragment suitable for use in the present invention.

Nucleic acid sequences encoding antibodies or antibody fragments for use with the present invention can be readily prepared by the skilled person. These techniques include (i) the use of the polymerase chain reaction (PCR) to amplify samples of such nucleic acid, e.g. from genomic sources, (ii) chemical synthesis, or (iii) preparing cDNA sequences. DNA encoding antibody fragments may be generated and used in any suitable way known to those of skill in the art, including by taking encoding DNA, identifying suitable restriction enzyme recognition sites either side of the portion to be expressed, and cutting out said portion from the DNA. The portion may then be operably linked to a suitable promoter in a standard commercially available expression system. Another recombinant approach is to amplify the relevant portion of the DNA with suitable PCR primers. Modifications to the sequences can be made, e.g. using site directed mutagenesis, to lead to the expression of modified peptide or to take account of codon preferences in the host cells used to express the nucleic acid.

The nucleic acid may be comprised as constructs in the form of a plasmid, vector, transcription or expression cassette which comprises at least one nucleic acid as described above. The construct may be comprised within a recombinant host cell which comprises one or more constructs as above. Expression may conveniently be achieved by culturing under appropriate conditions recombinant host cells containing the nucleic acid. Following production by expression the antibody or antibody fragments may be isolated and/or purified using any suitable technique, then used as appropriate.

Systems for cloning and expression of a polypeptide in a variety of different host cells are well known. Suitable host cells include bacteria, mammalian cells, yeast, insect and baculovirus systems. Mammalian cell lines available in the art for expression of a heterologous polypeptide include Chinese hamster ovary (CHO) cells, HeLa cells, baby hamster kidney cells, NS0 mouse myeloma cells. A common, preferred bacterial host is *E*. *coli.* The expression of antibodies and antibody fragments in prokaryotic cells such as *E*. *coli* is well established in the art. Expression in eukaryotic cells in culture is also available to those skilled in the art as an option for production of a binding member. General techniques for the production of antibodies are well known to the person skilled in the field.

Recombinant nucleic acids may comprise an insert coding for a heavy chain variable domain and/or for a light chain variable domain of an antibody. By definition such nucleic acids may comprise coding single stranded nucleic acids, double stranded nucleic acids consisting of said coding nucleic acids and of complementary nucleic acids thereto, or these complementary (single stranded) nucleic acids themselves.

Furthermore, nucleic acids encoding a heavy chain variable domain and/or a light chain variable domain of antibodies can be enzymatically or chemically synthesised nucleic acids having the authentic sequence coding for a naturally-occurring heavy chain variable domain and/or for the light chain variable domain, or a mutant thereof.

Recombinant DNA technology may be used to improve the antibodies. Thus, chimeric antibodies may be constructed in order to decrease the immunogenicity thereof in diagnostic or therapeutic applications. Moreover, immunogenicity within, for example, a transgenic organism such as a pig, may be minimised, by altering the antibodies by CDR grafting in a technique analogous to humanising antibodies. In order to reduce immunogenicity within a human recipient, the invention may employ recombinant nucleic acids comprising an insert coding for a heavy chain variable domain of an antibody fused to a human constant domain. Likewise recombinant DNAs comprising an insert coding for a light chain variable domain of an antibody fused to a human constant domain kappa or lambda region can be utilised with the present invention.

Antibodies may moreover be generated by mutagenesis of antibody genes to produce 5 artificial repertoires of antibodies. This technique allows the preparation of antibody libraries. Antibody libraries are also available commercially. Hence, the present invention advantageously employs artificial repertoires of immunoglobulins, preferably artificial scFv repertoires, as an immunoglobulin source in order to identify binding molecules which have specificity for the epitope described above.

### Antibody selection systems

Immunoglobulins which are able to bind to an epitope of TLR2 and which accordingly may be used in the methods of the invention can be identified using any technique known to the skilled person. Such immunoglobulins may be conveniently isolated from libraries comprising artificial repertoires of immunoglobulin polypeptides. A "repertoire" refers to a set of molecules generated by random, semi-random or directed variation of one or more template molecules, at the nucleic acid level, in order to provide a multiplicity of binding specificities. Methods for generating repertoires are well characterised in the art.

Selection protocols for isolating desired members of large libraries are known in the art, as typified by phage display techniques. Such systems, in which diverse peptide sequences are displayed on the surface of filamentous bacteriophage, have proven useful for creating libraries of antibody fragments (and the nucleotide sequences that encode them) for the *in-vitro* selection and amplification of specific antibody fragments that bind a target antigen. The nucleotide sequences encoding the VH and VL regions are linked to gene fragments which encode leader signals that direct them to the periplasmic space of *E*. *coli* and as a result the resultant antibody fragments are displayed on the surface of the bacteriophage, typically as fusions to bacteriophage coat proteins (for example pill or pVIII). Alternatively, antibody fragments are displayed externally on lambda phage capsids (phage bodies). An advantage of phage-based display systems is that, because they are biological systems, selected library members can be amplified simply by growing the phage containing the selected library member in bacterial cells. Furthermore, since the nucleotide sequence that encodes the polypeptide library member is contained on a phage or phagemid vector, sequencing, expression and subsequent genetic manipulation is relatively straight forward.

Methods for the construction of bacteriophage antibody display libraries and lambda phage expression libraries are well known in the art.

An alternative to the use of phage or other cloned libraries is to use nucleic acid, preferably RNA, derived from the B cells of an animal which has been immunised with the selected target, e.g. the TLR2 epitope described above.

Isolation of V-region and C-region mRNA permits antibody fragments, such as Fab or Fv, to be expressed intracellularly. Briefly, RNA is isolated from the B cells of an immunised animal, for example from the spleen of an immunised mouse or the circulating B cells of a llama, and PCR primers used to amplify VH and VL cDNA selectively from the RNA pool. The VH and VL sequences thus obtained are joined to make scFv antibodies. PCR primer sequences may be based on published VH and VL sequences.

### Peptidomimetics

Peptide analogues, such as peptidomimetics or peptide mimetics are non-peptide compounds with properties representative of a template peptide. Such peptide analogues are typically developed using computerised molecular modelling. Peptidomimetics which are structurally similar to peptides which have affinity and binding specificity to the TLR2 binding epitope described above may be used to mediate similar diagnostic, prophylactic and therapeutic effects.

Peptidomimetics are typically structurally similar to a template peptide, but have one or more peptide linkages replaced by an alternative linkage, by methods which are well known in the art. For example, a peptide which has a binding specificity for the TLR2 epitope described above may be modified such that it comprises amide bond replacement, incorporation of non peptide moieties, or backbone cyclisation. Suitably if cysteine is present the thiol of this residue is capped to prevent damage of the free sulphate group. A peptide may further be modified from the natural sequence to protect the peptides from protease attack.

Suitably a peptide described herein may be further modified using at least one of C and/or N-terminal capping, and/or cysteine residue capping. Suitably, a peptide may be capped at the N terminal residue with an acetyl group. Suitably, a peptide for use in the present invention may be capped at the C terminal with an amide group. Suitably, the thiol groups of cysteines are capped with acetamido methyl groups.

Expression, isolation and purification of polypeptides defining the epitope of TLR2 and fragments thereof may be accomplished by any suitable technique. A method for producing polypeptides comprises culturing host cells transformed with a recombinant expression vector encoding a polypeptide under conditions that promote expression of the polypeptide, then recovering the expressed polypeptides from the culture. The person skilled in the art will recognise that the procedure for purifying the expressed polypeptides will vary according to such factors as the type of host cells employed, and whether the polypeptide is intracellular, membrane-bound or a soluble form that is secreted from the host cell.

Any suitable expression system may be employed. The vectors include a DNA encoding a polypeptide or fragment of the invention, operably linked to suitable transcriptional or translational regulatory nucleotide sequences, such as those derived from a mammalian, avian, microbial, viral, bacterial, or insect gene. Nucleotide sequences are operably linked when the regulatory sequence functionally relates to the DNA sequence. Thus, a promoter nucleotide sequence is operably linked to a DNA sequence if the promoter nucleotide sequence controls the transcription of the DNA sequence. An origin of replication that confers the ability to replicate in the desired (*E.coli*) host cells, and a selection gene by which transformants are identified, are generally incorporated into the expression vector.

In addition, a sequence encoding an appropriate signal peptide (native or heterologous) can be incorporated into expression vectors. A DNA sequence for a signal peptide (secretory leader) may be fused in frame to the nucleic acid sequence of the invention so that the DNA is initially transcribed, and the mRNA translated, into a fusion protein comprising the signal peptide. A signal peptide that is functional in the intended host cells promotes extracellular secretion of the polypeptide. The signal peptide is cleaved from the polypeptide during translation, but allows secretion of polypeptide from the cell.

Suitable host cells for expression of polypeptides include higher eukaryotic cells and yeast. Prokaryotic systems are also suitable. Mammalian cells, and in particular CHO cells are particularly preferred for use as host cells.

### Small Molecules

A substance identified as an antagonist of the TLR2 receptor may be a peptide or may be non-peptide in nature, for example a peptidomimetic as described hereinbefore. However, non-peptide "small molecules" are often preferred for many *in-vivo* pharmaceutical uses. Accordingly, a mimetic or mimic of a TLR2 binding compound may be designed for use in a method described herein.

The designing of mimetics to a known pharmaceutically active compound is a known approach to the development of pharmaceuticals based on a "lead" compound. This might be desirable where the active compound is difficult or expensive to synthesise, or where it is unsuitable for a particular method of administration. For example, peptides are not well suited as active agents for oral compositions and administration as they are degraded by proteases present in the alimentary canal. Mimetic design, synthesis and testing may be used to avoid randomly screening large number of molecules for a target property.

There are several steps commonly taken in the design of a mimetic from a compound having a given target property. Firstly, the particular parts of the compound that are critical and/or important in determining the target property are determined. In the case of a peptide, this can be done by systematically varying the amino acid residues in the peptide, for example by substituting each amino acid residue in turn. These parts or residues constituting the active region of the compound are known as its "pharmacophore".

Once the pharmacophore has been determined, its structure is modelled according to its physical properties, e.g. stereochemistry, bonding, size and/or charge, using data from a range of sources, e.g. spectroscopic techniques, X-ray diffraction data and NMR. Computational analysis, similarity mapping (which models the charge and/or volume of a pharmacophore, rather than the bonding between atoms) and other techniques can also be used in this modelling process.

In a variant of this approach, the three-dimensional structure of the TLR2 binding compound is modelled. This can be especially useful where the ligand and/or binding partner change conformation on binding, allowing the model to take account of the design of the mimetic.

A template molecule is then selected onto which chemical groups which mimic the pharmacophore can be grafted. The template molecule and the chemical groups grafted on to it can conveniently be selected so that the mimetic is easy to synthesise, is likely to be pharmacologically acceptable, and does not degrade *in-vivo,* while retaining the biological activity of the lead compound. The mimetic or mimetics found by this approach can then be screened to see whether they have the target property, or to what extent they exhibit it. Further optimisation or modification can then be carried out to arrive at one or more final mimetics for *in-vivo* or clinical testing.

The mimetic binding compound may be a natural or synthetic chemical compound used in drug screening programmes. Extracts of plants which contain several characterised or uncharacterised components may also be used.

Combinatorial library technology provides an efficient way of testing a potentially vast number of different substances for ability their ability to bind to an epitope or to modulate the activity of a ligand which binds to an epitope. Prior to, or as well as, being screened for modulation of activity, test substances may be screened for ability to interact with the polypeptide, e.g. in a yeast two-hybrid system (which requires that both the polypeptide and the test substance can be expressed in yeast from encoding nucleic acid). This may be used as a coarse screen prior to testing a substance for actual ability to modulate activity of the polypeptide.

The amount of test substance or compound which may be added to an assay will normally be determined by trial and error depending upon the type of compound used. Typically, from about 0.01 to 100 nM concentrations of putative inhibitor compound may be used, for example from 0.1 to 10 nM. Greater concentrations may be used when a peptide is the test substance.

### Administration

The TLR2 antagonist may be administered alone, but will preferably be administered as a pharmaceutical composition, which will generally comprise a suitable pharmaceutically acceptable excipient, diluent or carrier selected depending on the intended route of administration. Examples of suitable pharmaceutical carriers include water, glycerol, ethanol and other GRAS reagents.

The TLR2 antagonist may be administered to a subject in need of treatment via any suitable route. As detailed herein, it is preferred that the composition is administered parenterally by injection or infusion. Examples of preferred routes for parenteral administration include, but are not limited to, intravenous, intracardial, intraarterial, intraperitoneal, intramuscular, intracavity, subcutaneous, transmucosal and transdermal. The composition can be administered by inhalation. Routes of administration may further include topical and enteral, for example, mucosal (including pulmonary), oral, nasal, rectal.

The composition may be delivered as an injectable composition. For intravenous, intramuscular, intradermal or subcutaneous application, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as sodium chloride injection, Ringer's injection or, Lactated Ringer's injection. Preservatives, stabilisers, buffers, antioxidants and/or other additives may be included, as required.

The composition may also be administered via microspheres, liposomes, other microparticulate delivery systems or sustained release formulations placed in certain tissues including blood.

The actual dose administered, and rate and time-course of administration, will depend on, and can be determined with due reference to, the nature and severity of the condition which is being treated, as well as factors such as the age, sex and weight of the subject to be treated and the route of administration. Further due consideration should be given to the properties of the composition, for example, its binding activity and in-vivo plasma life, the concentration of the antagonist in the formulation, as well as the route, site and rate of delivery.

Dosage regimens can include a single administration of the composition of the invention, or multiple administrative doses of the composition. The compositions can further be administered sequentially or separately with other therapeutics and medicaments which are used for the treatment of Myelodysplastic Syndrome.

The composition may be administered as a single dose or as repeated doses. Examples of dosage regimens which can be administered to a subject can be selected from the group comprising, but not limited to, 1µg/kg/day through to 20mg/kg/day, 1µg/kg/day through to 10mg/kg/day, 1mg/kg/day through to 15mg/kg/day, in the range of 2.5mg/kg/day to 15 mg/kg/day. In one embodiment, the daily dose is about 5mg/kg to 15mg/kg. In one embodiment, the daily dose is 10mg/kg.

The TLR2 antagonist may be orally administered to the subject, for example, at a dose of from about 1 mg/kg to about 10 mg/kg of the subject's body weight per day. Optionally the dose of the TLR2 antagonist is 5mg/kg. Optionally the dose of the TLR2 antagonist is 10mg/kg. Typically, the TLR2 antagonist is administered to the subject for a time and under conditions sufficient to down regulate the level and/or activity of TLR2.

Optionally, subjects can receive a starting dose of 5 mg/kg of a TLR2 antagonist as monotherapy. Treatment may continue with the dose remaining the same or increased to 10 mg/kg or 20 mg/kg. The dose can also potentially be decreased to 1.5 mg/kg or the frequency reduced from 4-weekly to 8-weekly. Each subject may undergo an efficacy evaluation and if a complete response/remission is seen in an individual subject in terms of cytological response or need for transfusions they may continue to receive OPN-305 as monotherapy. If an individual subject is not considered to have responded adequately they may receive a hypomethylating agent such as AZA in addition to OPN-305.

### Candidate Compounds

Also described herein is an assay method for identifying compounds for use in the treatment of Myelodysplastic Syndrome. The method comprises identifying compounds which bind to the same epitope on TLR2 as that bound by the T2.5 and OPN-305 antibodies.

Optionally the candidate compound is selected from the group consisting of a peptide, for example an antibody or antibody fragment, a chemical compound and a peptidomimetic. Optionally the candidate compound is a natural or synthetic chemical compound used in a drug screening programmes. Extracts of plants which contain several characterised or uncharacterised components may be used.

The precise format of the candidate compound screening assay may be varied by those skilled in the art using routine skill and knowledge. Combinatorial library technology provides an efficient way of testing a potentially vast number of different substances for the ability to bind to an epitope. The amount of candidate compound which may be added to an assay will normally be determined by trial and error depending upon the type of compound used. Typically, from about 0.01 to 100 nM concentrations of the candidate compound may be used, for example from 0.1 to 10 nM. Greater concentrations may be used when the candidate binding compound is a peptide. A candidate compound which has affinity and binding specificity for the binding epitope described herein may be isolated and/or purified, manufactured and/or used to modulate TLR2 functional activity in the treatment of Myelodysplastic Syndrome.

The present invention will now be described with reference to the following examples which are provided for the purpose of illustration and are not intended to be construed as being limiting on the present invention.

### Example

The inventors carried out a two-part, single centre, non-randomised, Phase I/II study that evaluated the safety, tolerability and efficacy of OPN-305 in patients with lower-risk (low and intermediate-1) MDS patients for who previous treatment with or without erythroid-stimulating agents or azacitidine or decitabine failed.

### METHODS

The inventors designed a phase I/II clinical trial of OPN-305 for patients with Low or Intermediate-1 risk MDS as characterised by IPSS after failure to prior therapy with a HMA. Patients with isolated del(5q) had received therapy with lenalidomide. Patients with prior history of AML or allogeneic hematopoietic stem cell transplantation (alloSCT) were excluded from the study. Because, OPN-305 had not been previously used in patients with hematological malignancies, the study had an initial phase of N=10 patients using OPN-305 at a dose of 5 mg/kg every 4 weeks for a maximum of 9 cycles. Therapy was repeated as long as there was no excess toxicity or progression. If after 16 weeks of therapy, there was no response, azacitidine on a 3 day schedule, was added to OPN-305. Responses were evaluated following the revised 2006 IWG criteria. This initial cohort allowed evaluation of toxicity, pharmacokinetic analysis, receptor occupancy, and sequential analysis of cytokine profile. Depending on results, dose escalation to 10 mg/kg monthly were instituted in a second cohort of N=5 pts.

A total of 15 patients were enrolled, 11 at the initial 5 mg dose and 4 at 10 mg/kg.

All patients were evaluable for toxicity and 12 (80%) were evaluable for response.

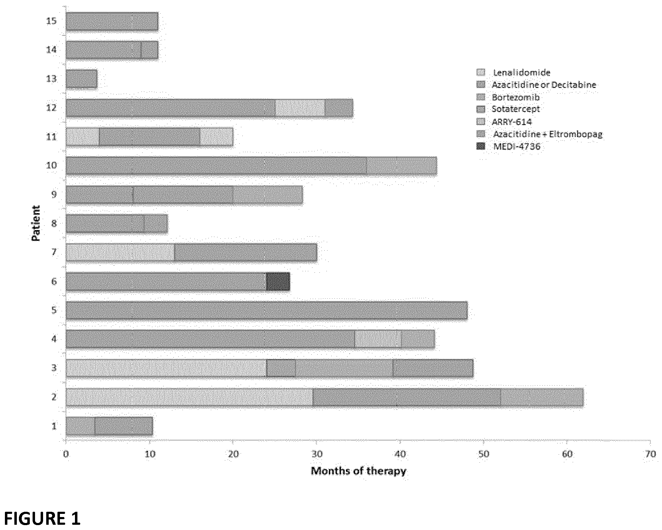

Patient characteristics are shown in Table 1. Median age was 73 years (range 48-87). Three (20%) patients were classified as Low risk and 12 (80%) as Intermediate-1 risk by IPSS. Seven patients had normal karyotype, 2 del(5q), 2 trisomy 8, 1 del(20q), 1 monosomy Y and 2 other single or double abnormalities. Median number of prior therapies was 2 (range 1-4) with a median duration of prior therapies of 28 months (range 4-62) (Figure 1).

A total of 13 (87%) patients were transfusion dependent of red cells prior to enrolment.

**Table 1: WBC = white blood cell count. ANC = absolute neutrophil count. MDS-SLD = MDS with single leneage dusplasia. MDS-RS = MDS with ringed sideroblasts. MDS-MLD = MDS with multiple lineage dysplsia. MDS-EB = MDS with excess blasts. MDS-U = MDS unclassifiable. IPSS = International Prognostic Scoring System. IPSS-R = Revised International Prognostic Scoring System.**

| **Variable** | | **Total Population (n=15) n (%) / mean [range or SD]** |
|---|---|---|
| **Age (years)** | | 73 [48-87] |
| **Male** | | 11 (73) |
| **WBC [x10⁹/L]** | | 3.8 [2.9-4.8] |
| **Hgb(g/dL)** | | 9.3 [8.4-10.3] |
| **Platelets (x10⁹/L)** | | 237 [123-351] |
| **ANC (x10⁹/L)** | | 1.9 [1.4-2.4] |
| **Bone marrow blasts (%)** | | 2 [0-6] |
| **LDH (IU/L)** | | 541 [459-624] |
| **Bilirubin (mg/dL)** | | 0.78 [0.54-1.02] |
| **Creatinine (mg/dL)** | | 0.88 [0.73-1.03] |

| **WHO Diagnosis** | | |
|---|---|---|
| | MDS-5LD | 2(13) |
| | MDS-RS | 2 (13) |
| | MDS-MLD | 3 (20) |
| | MD5-EB | 7 (47) |
| | MDS with del(5q) | 1 (7) |

| **IPSS** | | |
|---|---|---|
| | Low | 3 (20) |
| | Intermediate-1 | 12 (80) |

| **IPSS-R** | | |
|---|---|---|
| | Very Low | 1 (7) |
| | Low | 7 (47) |
| | Intermediate | 1 (7) |
| | High | 4 (27) |
| | Very high | 2 (13) |

| **IPSS-R Cytogenetic Risk Category** | | |
|---|---|---|
| | Very good | 2(13) |
| | Good | 9 (60) |
| | Intermediate | 4 (27) |
| | High | 0 (0) |
| | Very high | 0 (0) |

| **Prior lines of therapy** | | |
|---|---|---|
| | 1 | 3 (20) |
| | 2 | 6 (40) |
| | 3 | 5 (33) |
| | 4 | 1 (7) |

### RESULTS

Median follow up was 8 months. Median number of cycles administered was 5 (patients 2-17) with 5 patients (33%) having received addition of azacitidine after 16 weeks of therapy. A total of 5 patients (42%) developed Adverse Events (AE) related to OPN-305. All AEs were grade 1 with gastrointestinal disorders being the most frequent (33%). No significant drug related toxicity has been documented with no excess infectious complications.

In the first cohort of the study, patients received 5 mg/kg of OPN-305. 5/10 eligible patients had a response; one major response and 4 minor responses.

In the second phase of the study, patients received 10 mg/kg of OPN-305. Of the eligible patients who have received more than 2 cycles of OPN-305 monotherapy, 2/5 patients had a major response, 2/5 have stable disease and 1/5 had a minor response.

### Definition of Erythroid Response (HI-E)

### Major response:

- For patients with pre-treatment haemoglobin less than 11 g/dL, greater than 2 g/dL increase in haemoglobin;
- For RBC transfusion-dependent patients, transfusion independence

### Minor response:

- For patients with pre-treatment haemoglobin less than 11 g/dL, 1 to 2 g/dL increase in haemoglobin;
- For RBC transfusion-dependent patients, 50% decrease in transfusion requirements

### Stable disease

- Stable disease is defined as per the IWG 06 criteria: no progression in <8 weeks.

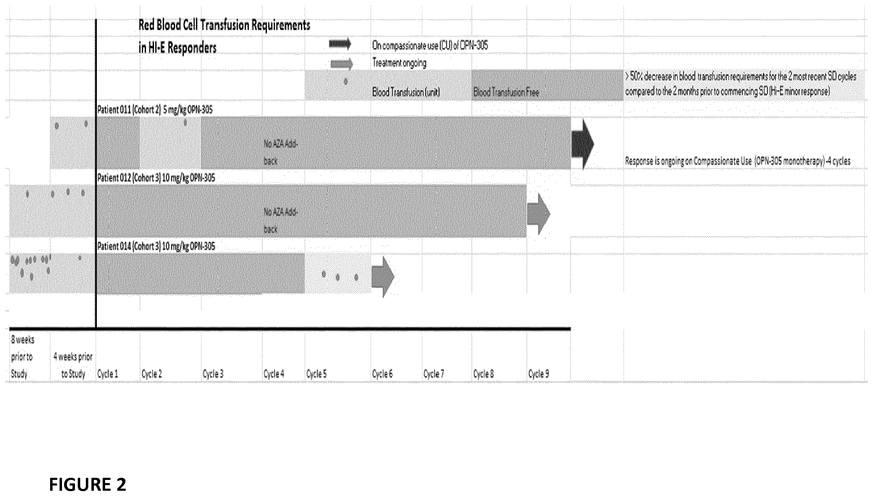

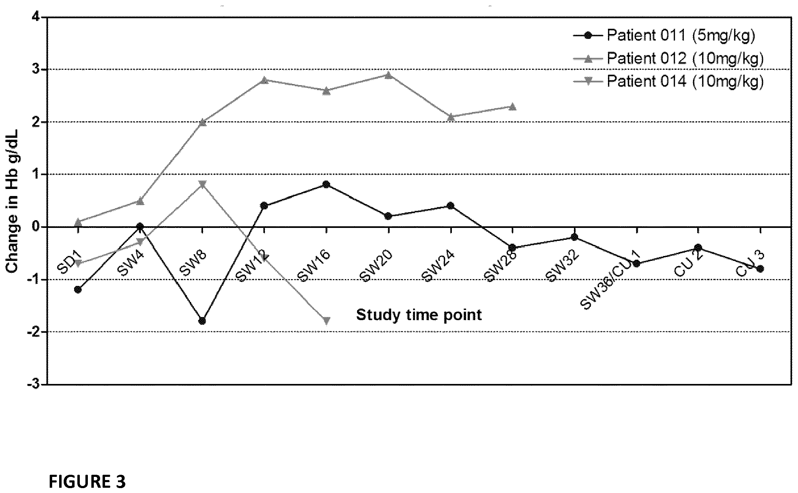

As illustrated in Figure 2, three patients are currently red blood cell transfusion independent and furthermore are maintaining or showing an increase in haemoglobin levels (Figure 3). In the case of these patients a major haematological response as per IWG 06 criteria has been noted with OPN-305 treatment as a monotherapy. In the case of two patients, a minor haematological response as per IWG 06 criteria has been noted with a combination treatment of OPN-305 and azacitidine.

### The overall response in evaluable patients (n=15) to date is as follows:

- 3/15 (20%) major response (transfusion independent, in all 3 patients OPN-305 treatment as monotherapy)
- 5/15 (33.3%) minor response *(2*/*5 OPN-305 and AZA* as *combination*, *3*/*5 OPN-305* as *monotherapy)*
- 6/15 (40%) Stable disease *(3*/*5 OPN-305 and AZA* as *combination 3*/*5 OPN-305* as *monotherapy)*

### Overall response rate (major & minor): 8/15 (53%)

Longest duration of response out to 8 cycles for patients on study with 18 cycles on compassionate use.

There was a greater-than-dose proportional increase in mean OPN-305 exposure (AUC) between 5 and 10 mg/kg. PK profiles after repeated dosing at 5 mg/kg in N=2 subjects and pre-dose (trough) levels in other subjects indicated some variability in the potential for accumulation. TLR-2 receptor occupancy in blood PBMCs and bone marrow aspirates was essentially complete in virtually all samples taken after OPN-305 administration. There is no evidence of treatment related anti-drug antibodies. Compared with baseline, no statistically significant dynamic changes of IL-23, IL-18, IFN-r, IL-10, IL-1β, IL-6, IL-12 (p40), IL-12 (p70) and IL-8 levels where observed after treatment, among responders or non-responders or based on OPN-305 dosing.

### TLR2 Blood Receptor Occupancy After Single and Repeat Dosing of OPN-305

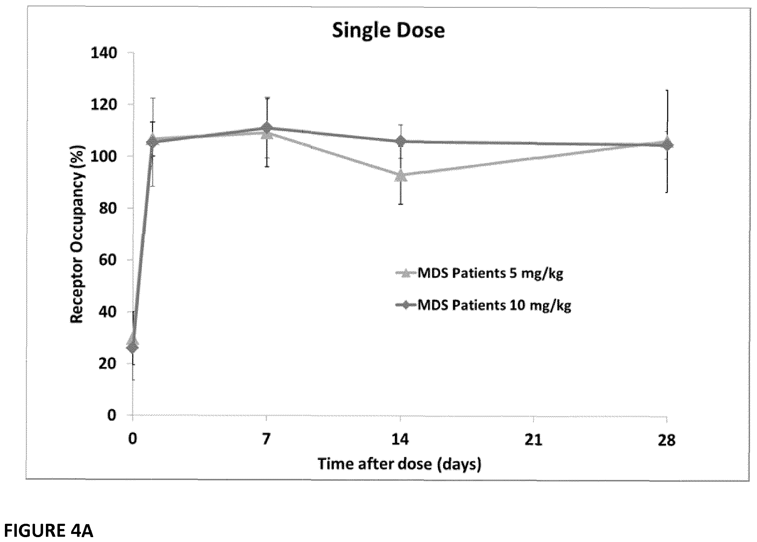

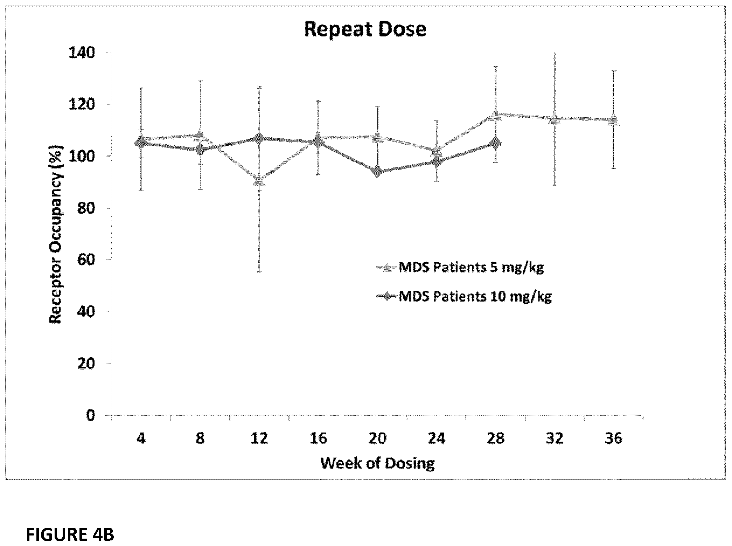

The TLR2 blood receptor occupancy after single and repeat dosing with the TLR2 antibody OPN-305 was determined. Full receptor occupancy was maintained for 28 days after a single IV dose at 5mg/kg or 10mg/kg (Figure 4A). These results were consistent with results at the same doses in healthy volunteers. At trough (pre-dose) upon repeat IV dose every four weeks (Figure 4B). There was no evidence of treatment-related anti-drug antibodies.

### TLR2 Bone Marrow Receptor Occupancy After Repeat Dosing of OPN-305

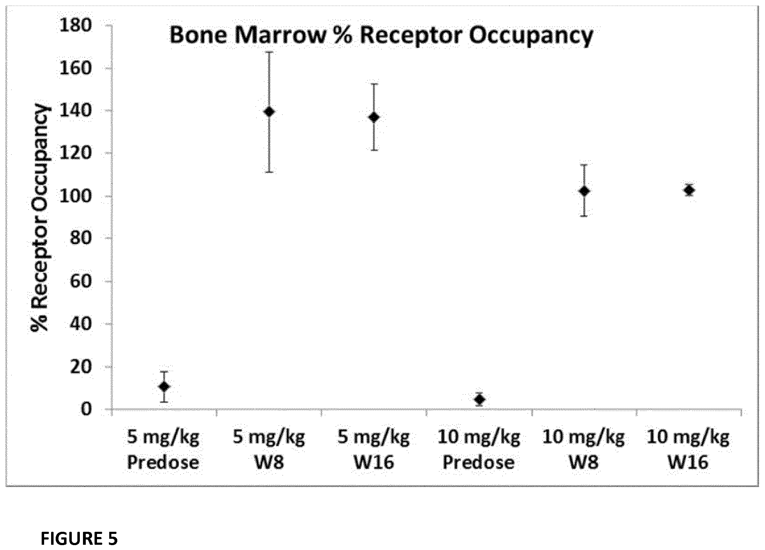

The TLR2 bone marrow receptor occupancy after repeat dosing with the TLR2 antibody OPN-305 was determined. Full receptor occupancy was demonstrated at week 8 and 16 at a dose of both 5mg/kg and 10mg/kg (Figure 5).

### Serum OPN-305 Concentrations After Single and Repeat Dosing

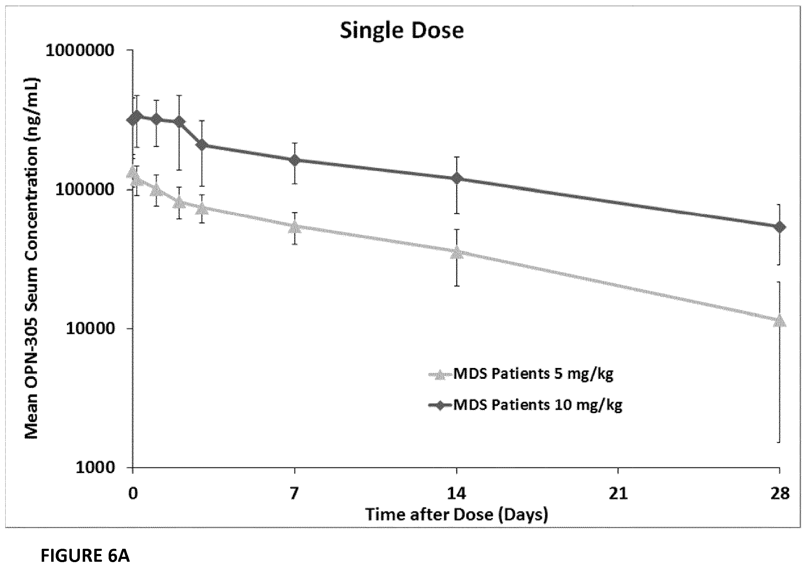

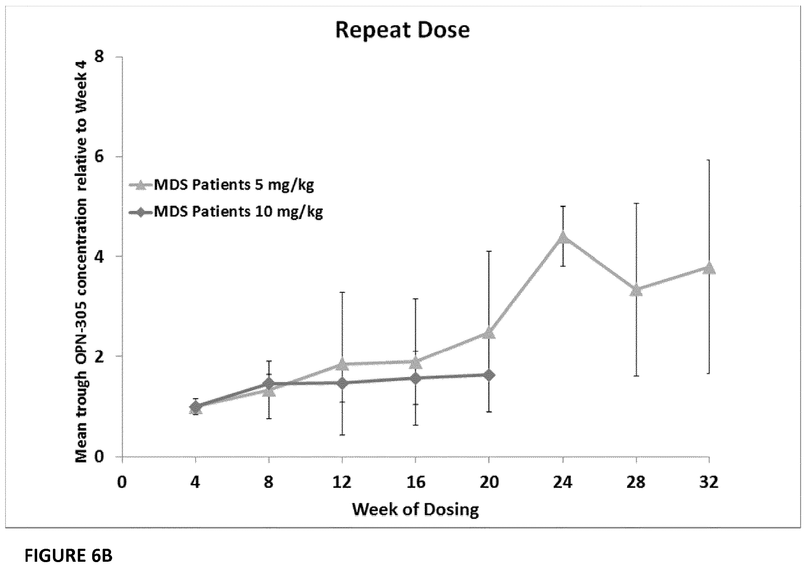

Serum concentrations of the TLR2 antibody OPN-305 after single and repeat dosing was determined. Mean dose-normalised exposure at 10mg/kg is double that seen at 5mg/kg (Figure 6A). This greater-than-dose proportionality is also seen in healthy volunteers. This is likely to reflect faster clearance at lower concentrations. Trough (pre-dose) serum concentrations (relative to week four) increased with repeat dosing at 5mg/kg. Individual subjects trough values stayed above serum concentrations and are expected to elicit full receptor occupancy (Figure 6B).

### The effect of expression levels of TLR2 in subjects

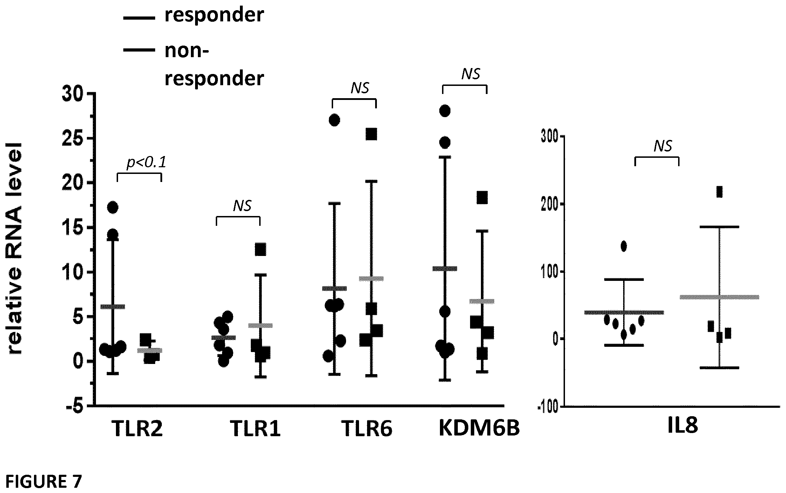

There is increased expression of TLR2 in patients who responded to OPN-305 treatment compared to patients who did not respond. The relative RNA level of TLR2 is increased in subjects who responded to treatment versus subjects who did not respond to treatment (Figure 7).

### CONCLUSION

Treatment with OPN-305 in patients with previously treated lower-risk MDS was well tolerated with no significant toxicities and 50% overall response rate. Correlative studies suggest adequate receptor occupancy with no correlation between responses and cytokine levels.

At 5 mg/kg 5/10 eligible patients have had a response (one major and 4 minor responses) and currently at 10 mg/kg, of the patients who have received more than 2 cycles of OPN-305 monotherapy, 2/5 eligible patients have had a major response), 2/5 stable disease and 1/5 minor response.

Modifications and variations to the described embodiments of the inventions will be apparent to those skilled in the art, without departing from the scope of the claims. Although the invention has been described in connection with specific preferred embodiments, it should be understood that the invention as claimed should not be unduly limited to such specific embodiments.

## Claims

1. A Toll-like receptor 2 (TLR2) antagonist for use in the treatment of lower-risk Myelodysplastic Syndrome in a subject who is refractory or resistant to a previous treatment with a hypomethylating agent, wherein the TLR2 antagonist is an antibody or an antigen binding fragment thereof that specifically binds to TLR2, wherein the antibody or an antigen binding fragment thereof specifically binds to a noncontinuous epitope comprising amino acid residues His318, Pro320, Arg321, Tyr323, Lys347, Phe349, Leu371, Glu375, Tyr376 and His398 of SEQ ID NO: 1 or His318, Pro320, Gln321, Tyr323, Lys347, Phe349, Leu371, Glu375, Tyr376 and His398 of SEQ ID NO: 2, and wherein said treatment of Myelodysplastic Syndrome is a combination treatment with a therapeutically effective amount of a secondary therapeutic agent which is administered sequentiallyor simultaneously with said TLR2 antagonist, wherein the secondary therapeutic agent is a hypomethylating agent.

2. The TLR2 antagonist for use of claim 1, wherein the hypomethylating agent is azacitidine or decitabine.

3. The TLR2 antagonist for use as claimed in claim 1 or claim 2, wherein the antibody or antigen binding fragment thereof specifically binds to an epitope comprising leucine rich repeat regions 11 to 14 of TLR2.

4. The TLR2 antagonist for use as claimed in any one of claims 1 to 3, wherein the antibody or antigen binding fragment comprises a heavy chain variable region comprising a complementarity determining region (CDR) 1 region comprising the amino acid sequence of SEQ ID NO:3, a CDR2 region comprising the amino acid sequence of SEQ ID NO:4 and a CDR3 region comprising the amino acid sequence of SEQ ID NO:5, and a light chain variable region comprising a CDR1 region comprising the amino acid sequence of SEQ ID NO:6, a CDR2 region comprising the amino acid sequence Gly-Ala-Ser and a CDR3 region comprising the amino acid sequence of SEQ ID NO:7.

5. The TLR2 antagonist for use as claimed in claim 4, wherein the antibody or antigen binding fragment comprises a light chain variable domain comprising
an amino acid sequence of SEQ ID NO:10, or a sequence which has at least 90% amino acid sequence identity with SEQ ID NO:10, and/or a heavy chain variable domain comprising an amino acid sequence of SEQ ID NO:11, or a sequence which has at least 90% amino acid sequence identity with SEQ ID NO:11.

6. The TLR2 antagonist for use as claimed in any one of claims 1 to 4, wherein the antibody comprises a heavy chain comprising the amino acid sequence of SEQ ID NO:13, or a sequence which has at least 90% amino acid sequence identity to the amino acid sequence of SEQ ID NO:13, and/or a light chain comprising the amino acid sequence of SEQ ID NO:12, or a sequence which has at least 90% amino acid sequence identity to the amino acid sequence of SEQ ID NO:12, or an antigen binding fragment thereof.

7. The TLR2 antagonist for use as claimed in any one of claims 1 to 4, wherein the antibody or antigen binding fragment comprises a heavy chain variable region comprising the amino acid sequence of SEQ ID NO:8, or a sequence which has at least 90% amino acid sequence identity to the amino acid sequence of SEQ ID NO:8, and/or a light chain variable region comprising the amino acid sequence of SEQ ID NO:9, or a sequence which has at least 90% amino acid sequence identity to the amino acid sequence of SEQ ID NO:9.

8. The TLR2 antagonist for use as claimed in any one of claims 1 to 6, wherein the antibody is a humanised version of anti-TLR2 antibody T2.5, or an antigen binding fragment thereof.

9. The TLR2 antagonist for use as claimed in any one of claims 1 to 8, wherein the secondary therapeutic is azacitidine.

10. The TLR2 antagonist for use as claimed in any one of claims 1 to 8, wherein the secondary therapeutic is decitabine.

11. The TLR2 antagonist for use as claimed in any one of claims 1 to 10, wherein the antibody or antigen binding fragment is administered to the subject at a dose of 10 mg/kg of the subject's body weight.

## Patentansprüche

1. Ein Toll-like-Rezeptor-2(TLR2)-Antagonist zur Verwendung bei der Behandlung eines myelodysplastischen Syndroms mit geringerem Risiko bei einem Individuum, das refraktär oder resistent gegen eine vorherige Behandlung mit einem hypomethylierenden Mittel ist, wobei der TLR2-Antagonist ein Antikörper oder ein antigenbindendes Fragment davon ist, das spezifisch an TLR2 bindet, wobei der Antikörper oder ein antigenbindendes Fragment davon spezifisch an ein nichtkontinuierliches Epitop bindet, beinhaltend die Aminosäurereste His318, Pro320, Arg321, Tyr323, Lys347, Phe349, Leu371, Glu375, Tyr376 und His398 von SEQ ID NO: 1 oder His318, Pro320, Gln321, Tyr323, Lys347, Phe349, Leu371, Glu375, Tyr376 und His398 von SEQ ID NO: 2, und wobei die Behandlung von myelodysplastischem Syndrom eine Kombinationsbehandlung mit einer therapeutisch wirksamen Menge eines sekundären therapeutischen Mittels ist, das sequenziell oder gleichzeitig mit dem TLR2-Antagonisten verabreicht wird, wobei das sekundäre therapeutische Mittel ein hypomethylierendes Mittel ist.

2. TLR2-Antagonist zur Verwendung gemäß Anspruch 1, wobei das hypomethylierende Mittel Azacitidin oder Decitabin ist.

3. TLR2-Antagonist zur Verwendung gemäß Anspruch 1 oder Anspruch 2, wobei der Antikörper oder das antigenbindende Fragment davon spezifisch an ein Epitop bindet, das leucinreiche Wiederholungsregionen 11 bis 14 von TLR2 beinhaltet.

4. TLR2-Antagonist zur Verwendung gemäß einem der Ansprüche 1 bis 3, wobei der Antikörper oder das antigenbindende Fragment Folgendes beinhaltet: eine variable Schwerkettenregion, die eine komplementaritätsbestimmende Region (CDR)-1-Region beinhaltet, die die Aminosäuresequenz von SEQ ID NO: 3 beinhaltet, eine CDR2-Region, die die Aminosäuresequenz von SEQ ID NO: 4 beinhaltet, und eine CDR3-Region, die die Aminosäuresequenz von SEQ ID NO: 5 beinhaltet, und eine variable Leichtkettenregion, die eine CDR1-Region beinhaltet, die die Aminosäuresequenz von SEQ ID NO: 6 beinhaltet, eine CDR2-Region, die die Aminosäuresequenz Gly-Ala-Ser beinhaltet, und eine CDR3-Region, die die Aminosäuresequenz von SEQ ID NO: 7 beinhaltet.

5. TLR2-Antagonist zur Verwendung gemäß Anspruch 4, wobei der Antikörper oder das antigenbindende Fragment Folgendes beinhaltet: eine variable Leichtkettendomäne, die eine Aminosäuresequenz von SEQ ID NO: 10 beinhaltet, oder eine Sequenz, die mindestens 90 % Aminosäuresequenzidentität mit SEQ ID NO: 10 aufweist, und/oder eine variable Schwerkettendomäne, die eine Aminosäuresequenz von SEQ ID NO: 11 beinhaltet, oder eine Sequenz, die mindestens 90 % Aminosäuresequenzidentität mit SEQ ID NO: 11 aufweist.

6. TLR2-Antagonist zur Verwendung gemäß einem der Ansprüche 1 bis 4, wobei der Antikörper Folgendes beinhaltet: eine schwere Kette, die die Aminosäuresequenz von SEQ ID NO: 13 beinhaltet, oder eine Sequenz, die mindestens 90 % Aminosäuresequenzidentität mit der Aminosäuresequenz von SEQ ID NO: 13 aufweist, und/oder eine leichte Kette, die die Aminosäuresequenz von SEQ ID NO: 12 beinhaltet, oder einer Sequenz, die mindestens 90 % Aminosäuresequenzidentität mit der Aminosäuresequenz von SEQ ID NO: 12 oder ein antigenbindendes Fragment davon aufweist.

7. TLR2-Antagonist zur Verwendung gemäß einem der Ansprüche 1 bis 4, wobei der Antikörper oder das antigenbindende Fragment Folgendes beinhaltet: eine variable Schwerkettenregion, die die Aminosäuresequenz von SEQ ID NO: 8 beinhaltet, oder eine Sequenz, die mindestens 90 % Aminosäuresequenzidentität mit der Aminosäuresequenz von SEQ ID NO: 8 aufweist, und/oder eine variable Leichtkettenregion, die die Aminosäuresequenz von SEQ ID NO: 9 beinhaltet, oder eine Sequenz, die mindestens 90 % Aminosäuresequenzidentität mit der Aminosäuresequenz von SEQ ID NO: 9 aufweist.

8. TLR2-Antagonist zur Verwendung gemäß einem der Ansprüche 1 bis 6, wobei der Antikörper eine humanisierte Version des Anti-TLR2-Antikörpers T2.5 oder ein antigenbindendes Fragment davon ist.

9. TLR2-Antagonist zur Verwendung gemäß einem der Ansprüche 1 bis 8, wobei das sekundäre Therapeutikum Azacitidin ist.

10. TLR2-Antagonist zur Verwendung gemäß einem der Ansprüche 1 bis 8, wobei das sekundäre Therapeutikum Decitabin ist.

11. TLR2-Antagonist zur Verwendung gemäß einem der Ansprüche 1 bis 10, wobei der Antikörper oder das antigenbindende Fragment dem Individuum in einer Dosis von 10 mg/kg des Körpergewichts des Individuums verabreicht wird.

## Revendications

1. Un antagoniste du récepteur de type Toll 2 (TLR2, *Toll-Like Receptor 2*) à utiliser dans le traitement du syndrome myélodysplasique de risque plus faible chez un sujet qui est réfractaire ou résistant à un traitement précédent avec un agent hypométhylant, où l'antagoniste du TLR2 est un anticorps ou un fragment de liaison à l'antigène de celui-ci qui se lie spécifiquement au TLR2, où l'anticorps ou un fragment de liaison à l'antigène de celui-ci se lie spécifiquement à un épitope discontinu comprenant des résidus d'acides aminés His318, Pro320, Arg321, Tyr323, Lys347, Phe349, Leu371, Glu375, Tyr376 et His398 de SEQ ID NO : 1 ou His318, Pro320, Gln321, Tyr323, Lys347, Phe349, Leu371, Glu375, Tyr376 et His398 de SEQ ID NO : 2, et où ledit traitement du syndrome myélodysplasique est un traitement d'association avec une quantité thérapeutiquement efficace d'un agent thérapeutique secondaire qui est administré séquentiellement ou simultanément avec ledit antagoniste du TLR2, où l'agent thérapeutique secondaire est un agent hypométhylant.

2. L'antagoniste du TLR2 à utiliser de la revendication 1, où l'agent hypométhylant est l'azacitidine ou la décitabine.

3. L'antagoniste du TLR2 à utiliser tel que revendiqué dans la revendication 1 ou la revendication 2, où l'anticorps ou le fragment de liaison à l'antigène de celui-ci se lie spécifiquement à un épitope comprenant les régions de répétition riche en leucine 11 à 14 du TLR2.

4. L'antagoniste du TLR2 à utiliser tel que revendiqué dans n'importe laquelle des revendications 1 à 3, où l'anticorps ou le fragment de liaison à l'antigène comprend une région variable de chaîne lourde comprenant une région déterminant la complémentarité (région CDR, *Complementarity Determining Region*) 1 comprenant la séquence d'acides aminés de SEQ ID NO : 3, une région CDR2 comprenant la séquence d'acides aminés de SEQ ID NO : 4 et une région CDR3 comprenant la séquence d'acides aminés de SEQ ID NO : 5, et une région variable de chaîne légère comprenant une région CDR1 comprenant la séquence d'acides aminés de SEQ ID NO : 6, une région CDR2 comprenant la séquence d'acides aminés Gly-Ala-Ser et une région CDR3 comprenant la séquence d'acides aminés de SEQ ID NO : 7.

5. L'antagoniste du TLR2 à utiliser tel que revendiqué dans la revendication 4, où l'anticorps ou le fragment de liaison à l'antigène comprend un domaine variable de chaîne légère comprenant une séquence d'acides aminés de SEQ ID NO : 10, ou une séquence qui présente une identité de séquence d'acides aminés d'au moins 90 % avec SEQ ID NO : 10, et/ou un domaine variable de chaîne lourde comprenant une séquence d'acides aminés de SEQ ID NO : 11, ou une séquence qui présente une identité de séquence d'acides aminés d'au moins 90 % avec SEQ ID NO : 11.

6. L'antagoniste du TLR2 à utiliser tel que revendiqué dans n'importe laquelle des revendications 1 à 4, où l'anticorps comprend une chaîne lourde comprenant la séquence d'acides aminés de SEQ ID NO : 13, ou une séquence qui présente une identité de séquence d'acides aminés d'au moins 90 % par rapport à la séquence d'acides aminés de SEQ ID NO : 13, et/ou une chaîne légère comprenant la séquence d'acides aminés de SEQ ID NO : 12, ou une séquence qui présente une identité de séquence d'acides aminés d'au moins 90 % par rapport à la séquence d'acides aminés de SEQ ID NO : 12, ou un fragment de liaison à l'antigène de celles-ci.

7. L'antagoniste du TLR2 à utiliser tel que revendiqué dans n'importe laquelle des revendications 1 à 4, où l'anticorps ou le fragment de liaison à l'antigène comprend une région variable de chaîne lourde comprenant la séquence d'acides aminés de SEQ ID NO : 8, ou une séquence qui présente une identité de séquence d'acides aminés d'au moins 90 % par rapport à la séquence d'acides aminés de SEQ ID NO : 8, et/ou une région variable de chaîne légère comprenant la séquence d'acides aminés de SEQ ID NO : 9, ou une séquence qui présente une identité de séquence d'acides aminés d'au moins 90 % par rapport à la séquence d'acides aminés de SEQ ID NO : 9.

8. L'antagoniste du TLR2 à utiliser tel que revendiqué dans n'importe laquelle des revendications 1 à 6, où l'anticorps est une version humanisée de l'anticorps anti-TLR2 T2.5, ou un fragment de liaison à l'antigène de celui-ci.

9. L'antagoniste du TLR2 à utiliser tel que revendiqué dans n'importe laquelle des revendications 1 à 8, où l'agent thérapeutique secondaire est l'azacitidine.

10. L'antagoniste du TLR2 à utiliser tel que revendiqué dans n'importe laquelle des revendications 1 à 8, où l'agent thérapeutique secondaire est la décitabine.

11. L'antagoniste du TLR2 à utiliser tel que revendiqué dans n'importe laquelle des revendications 1 à 10, où l'anticorps ou le fragment de liaison à l'antigène est administré au sujet à une dose de 10 mg/kg de poids corporel du sujet.
